# EUROPEAN PATENT APPLICATION

(11) **EP 3 770 256 A1**
(43) Date of publication of application: **27.01.2021**
(21) Application number: 19771001.5
(22) Date of filing: 20.03.2019
(51) Int. Cl.: C12N 15/113, A61K 31/712, A61K 31/7125, A61K 47/61, A61K 47/64, A61K 47/68, A61K 48/00, A61P 3/00, A61P 9/00, A61P 27/02, A61P 29/00, A61P 31/00, A61P 35/00, A61P 37/06

(54) **ANTISENSE OLIGONUCLEOTIDE HAVING REDUCED TOXICITY**

(30) Priority: 20.03.2018 JP 2018052578; 06.07.2018 JP 2018129296
(71) Applicant: Tokyo Institute of Technology, Tokyo 152-8550 (JP); Nissan Chemical Corporation, Tokyo 103-6119 (JP)
(72) Inventor: MASAKI, Yoshiaki, Tokyo 152-8550 (JP); SEIO, Kohji, Tokyo 152-8550 (JP); INOUE, Atsushi, Tokyo 152-8550 (JP); IRIYAMA, Yusuke, Funabashi-shi, Chiba 274-8507 (JP); KANAKI, Tatsuro, Shiraoka-shi, Saitama 349-0294 (JP); NAKAJIMA, Hiroyuki, Shiraoka-shi, Saitama 349-0294 (JP)
(74) Representative: dompatent von Kreisler Selting Werner - Partnerschaft von Patent- und Rechtsanwälten mbB
(86) International application number: PCT/JP2019/011801
(87) International publication number: WO 2019/182037

(57) **Abstract**

This is to provide an antisense oligonucleotide reduced in toxicity.

An antisense oligonucleotide having a central region, a 5'-side region and a 3'-side region, and the antisense oligonucleotide comprises the central region having a nucleotide (2'-3' bridged nucleotide) in which the 2'-position and the 3'-position of a sugar moiety are bridged and/or a non-bridged nucleotide (3'-position-modified non-bridged nucleotide) having a substituent at the 3'-position.

SEQUENCE LISTING

<110>  NISSAN CHEMICAL CORPORATION
       TOKYO INSTITUTE OF TECHNOLOGY

<120>  Antisense oligonucleotide with reduced toxicity

<130>  FP4404PCT

<150>  JP 2018-052578
<151>  2018-03-20

<150>  JP 2018-129296
<151>  2018-07-06

<160>  11

<170>  PatentIn version 3.5

<210>  1
<211>  16
<212>  DNA
<213>  Artificial Sequence

<220>
<223>  artificially synthesized oligonucleotide


<220>
<221>  modified_base
<222>  (1)..(3)
<223>  LNA

<220>
<221>  modified_base
<222>  (1)..(16)
<223>  phosphorothioate bond between nucleosides

<220>
<221>  modified_base
<222>  (9)..(9)
<223>  2'-O, 3'-C-bridged nucleioside

<220>
<221>  modified_base
<222>  (14)..(16)
<223>  LNA

<400>  1
tgaggtcctg cactgg                                                    16


<210>  2
<211>  16
<212>  DNA
<213>  Artificial Sequence

<220>
<223>  artificially synthesized oligonucleotide


<220>
<221>  modified_base

<222>  (1)..(3)
<223>  LNA

<220>
<221>  modified_base
<222>  (1)..(16)
<223>  phosphorothioate bond between nucleosides

<220>
<221>  modified_base
<222>  (14)..(16)
<223>  LNA

<400>  2
tgaggtcctg cactgg                                                    16


<210>  3
<211>  16
<212>  RNA
<213>  Artificial Sequence

<220>
<223>  artificially synthesized oligonucleotide


<220>
<221>  modified_base
<222>  (1)..(1)
<223>  oligonucleotide labeled with 6-FAM at 5'-end

<400>  3
ccagugcagg accuca                                                    16


<210>  4
<211>  16
<212>  DNA
<213>  Artificial Sequence

<220>
<223>  artificially synthesized oligonucleotide


<220>
<221>  modified_base
<222>  (1)..(3)
<223>  LNA

<220>
<221>  modified_base
<222>  (1)..(16)
<223>  phosphorothioate bond between nucleosides

<220>
<221>  modified_base
<222>  (3)..(3)
<223>  m5c

<220>
<221>  modified_base
<222>  (10)..(10)
<223>  2'-O,3'-C-bridged nucleoside

<220>
<221>  modified_base
<222>  (14)..(16)
<223>  LNA

<220>
<221>  modified_base
<222>  (15)..(16)
<223>  m5c

<400>  4
atctgtgcat ctctcc                                                        16


<210>  5
<211>  16
<212>  DNA
<213>  Artificial Sequence

<220>
<223>  artificially synthesized oligonucleotide


<220>
<221>  modified_base
<222>  (1)..(3)
<223>  LNA

<220>
<221>  modified_base
<222>  (1)..(16)
<223>  phosphorothioate bond between nucleosides

<220>
<221>  modified_base
<222>  (3)..(3)
<223>  m5c

<220>
<221>  modified_base
<222>  (14)..(16)
<223>  LNA

<220>
<221>  modified_base
<222>  (15)..(16)
<223>  m5c

<400>  5
atctgtgcat ctctcc                                                        16


<210>  6
<211>  16
<212>  RNA
<213>  Artificial Sequence

<220>
<223>  artificially synthesized oligonucleotide


<220>

<221>  modified_base
<222>  (1)..(1)
<223>  oligonucleotide labeled with 6-FAM at 5'-end

<400>  6
ggagagaugc acagau                                                    16


<210>  7
<211>  15
<212>  DNA
<213>  Artificial Sequence

<220>
<223>  artificially synthesized oligonucleotide


<220>
<221>  modified_base
<222>  (1)..(1)
<223>  2'-O-methoxyethyl-5-methyluridine

<220>
<221>  modified_base
<222>  (1)..(15)
<223>  phosphorothioate bond between nucleosides

<220>
<221>  modified_base
<222>  (2)..(3)
<223>  LNA

<220>
<221>  modified_base
<222>  (8)..(8)
<223>  2'-O,3'-C-bridged nucleoside

<220>
<221>  modified_base
<222>  (13)..(14)
<223>  LNA

<220>
<221>  modified_base
<222>  (14)..(14)
<223>  m5c

<220>
<221>  modified_base
<222>  (15)..(15)
<223>  2'-O-methoxyethyl-5-methylcytidine

<400>  7
taaattgtca tcacc                                                     15


<210>  8
<211>  15
<212>  DNA
<213>  Artificial Sequence

<220>
<223>  artificially synthesized oligonucleotide

<220>
<221>  modified_base
<222>  (1)..(1)
<223>  2'-O-methoxyethyl-5-methyluridine

<220>
<221>  modified_base
<222>  (1)..(15)
<223>  phosphorothioate bond between nucleosides

<220>
<221>  modified_base
<222>  (2)..(3)
<223>  LNA

<220>
<221>  modified_base
<222>  (13)..(14)
<223>  LNA

<220>
<221>  modified_base
<222>  (14)..(14)
<223>  m5c

<220>
<221>  modified_base
<222>  (15)..(15)
<223>  2'-O-methoxyethyl-5-methylcytidine

<400>  8
taaattgtca tcacc                                                        15


<210>  9
<211>  15
<212>  RNA
<213>  Artificial Sequence

<220>
<223>  artificially synthesized oligonucleotide


<220>
<221>  modified_base
<222>  (1)..(1)
<223>  oligonucleotide labeled with 6-FAM at 5'-end

<400>  9
ggugaugaca auuua                                                        15


<210>  10
<211>  15
<212>  RNA
<213>  Artificial Sequence

<220>
<223>  artificially synthesized oligonucleotide

<220>
<221>  modified_base
<222>  (1)..(1)
<223>  oligonucleotide labeled with 6-FAM at 5'-end

<400>  10
ggugauggca auuua                                                        15


<210>  11
<211>  16
<212>  DNA
<213>  Artificial Sequence

<220>
<223>  artificially synthesized oligonucleotide


<220>
<221>  modified_base
<222>  (1)..(3)
<223>  LNA

<220>
<221>  modified_base
<222>  (1)..(16)
<223>  phosphorothioate bond between nucleosides

<220>
<221>  modified_base
<222>  (3)..(3)
<223>  m5c

<220>
<221>  modified_base
<222>  (10)..(10)
<223>  3'-C-modified unbridged nucleoside

<220>
<221>  modified_base
<222>  (14)..(16)
<223>  LNA

<220>
<221>  modified_base
<222>  (15)..(16)
<223>  m5c

<400>  11
atctgtgcat ctctcc                                                       16

## Description

### TECHNICAL FIELD

The present invention relates to an antisense oligonucleotide reduced in toxicity.

### BACKGROUND ART

A nucleic acid medicine is a medicine comprising nucleic acids (oligonucleotides) that form complementary base pairs with a target DNA or RNA, and is expected as a novel medicine. And as nucleic acid units to be used for the nucleic acid medicines, various artificial nucleic acid units (artificial nucleosides or artificial nucleotides which are phosphoric acid adducts thereof) have been developed. For example, it has been known that by methoxyethylating (MOE) of an oxygen atom at the 2'-position of the sugar moiety of a ribonucleotide, affinity for a target nucleic acid, and resistance to a nuclease are improved (for example, see Patent Document 1). In addition, 2',4'-BNA and 2',4'-LNA are compounds in which the 2'-position and the 4'-position of the sugar moiety of a nucleic acid unit are bridged, and it has been known to have high affinity for the target nucleic acid (for example, see Patent Documents 2 to 5). Further, it has also been known nucleotides (2'-3' bridged nucleotide) in which the 2'-position and the 3'-position are bridged, or nucleotide (3'-position-modified non-bridged nucleotide) in which alkyl is introduced into the β-position at the 3'-position carbon atom of the sugar moiety. It has been investigated about the effects on the RNA strand-cleaving activity of RNase H by introducing these artificial nucleic acids into the DNA strand (for example, see Non-Patent Documents 1 and 2).

Development of gapmer type antisense nucleic acids in which artificial nucleic acid units are introduced into both ends of a single-stranded oligodeoxyribonucleotide is now progressing. It has been known that the gapmer type antisense nucleic acid forms a double-stranded complex with a target RNA, and RNase H in the cell recognizes the double-stranded portion of the deoxyribonucleotide portion and the target RNA and cleaves the RNA strand.

For applying the gapmer type antisense nucleic acids to medical practice, high sequence specificity is required. However, in recent years, toxicity caused by the off-target effect has been reported (for example, see Non-Patent Documents 3 and 4). The off-target effect occurs when a double-stranded complex by an antisense nucleic acid and RNA having a similar sequence other than the target is formed, and the RNA other than the target is cleaved. However, there is no report on a modification method for reducing such toxicity.

Also, in the case where a gene having a single nucleotide polymorphism (SNP) is targeted, selectivity of the mutant type to the wild type is required, and an investigation using an artificial nucleic acid in which the sugar moiety is modified by fluorine has been reported (for example, see Non-Patent Document 5).

### PRIOR ART DOCUMENTS

### PATENT DOCUMENTS

Patent Document 1: JP Hei.7-2889A
Patent Document 2: WO 98/39352
Patent Document 3: WO 2009/006478
Patent Document 4: WO 2011/052436
Patent Document 5: WO 2015/125783

### NON-PATENT DOCUMENTS

Non-Patent Document 1: Bioorganic & Medicinal Chemistry Letters, 2008, 18, pp. 2296-2300
Non-Patent Document 2: The Journal of Biological Chemistry, 2004, 279, pp. 36317-36326
Non-Patent Document 3: Nucleic Acids Research, 2016, 44, pp. 2093-2109
Non-Patent Document 4: Scientific Reports, 2016, 6, 30377
Non-Patent Document 5: Molecular Therapy - Nucleic Acids, 2017, 7, pp. 20-30

### DISCLOSURE OF THE INVENTION

### PROBLEMS TO BE SOLVED BY THE INVENTION

In gapmer type antisense nucleic acids, a novel technique which reduces toxicity caused by the "off-target effect" has been required.

Also, in the case where the single nucleotide polymorphism (SNP) portion is targeted, improvement in selectivity of the mutant type from the wild type has been required, but the sequence of the gapmer type antisense nucleic acids contain only a single base mismatch with the sequence of the wild type RNA, so that it is still difficult to obtain selectivity of the wild type/mutant type. Therefore, a novel technique for solving this problem has been required.

An object of the present invention is to provide an antisense oligonucleotide reduced in toxicity.

### MEANS FOR SOLVING THE PROBLEMS

The present inventors have found that a gapmer type antisense nucleic acid which has a nucleotide (2'-3' bridged nucleotide) in which the 2'-position and the 3'-position of the sugar moiety are bridged and/or a non-bridged nucleotide (3'-position-modified non-bridged nucleotide) having a substituent at the 3'-position, at the central region, is low toxicity, and has a high sequence selectivity, whereby they have accomplished the present invention. Incidentally, it has been reported that use of a part of the sugar moiety-modified nucleotides affects RNA strand cleavage activity by RNase H, but there is no report that these nucleotides reduce toxicity caused by the off-target effect, and there has been not reported about the relationship between control of the RNA strand cleavage activity by RNase H and reduction of toxicity caused by the off-target effect. It has been clarified by the present invention that toxicity caused by the off-target effect can be reduced by controlling the cleaved position. That is, the present invention includes the following embodiments.
1. An antisense oligonucleotide having a central region, a 5'-side region and a 3'-side region,
   wherein
   - the central region comprises
      at least 5 nucleotides independently selected from the group consisting of deoxyribonucleotides, ribonucleotides and sugar moiety-modified nucleotides, contains at least one sugar moiety-modified nucleotide selected from the group consisting of a 2'-3' bridged nucleotide and 3'-position-modified non-bridged nucleotide, and a 3'-terminal and a 5'-terminal thereof being each independently a deoxyribonucleotide, ribonucleotide, 2'-3' bridged nucleotide or 3'-position-modified non-bridged nucleotide, and
      contains at least one oligonucleotide strand constituted by at least four contiguous nucleotides which are independently selected from the group consisting of deoxyribonucleotides, 2'-3' bridged nucleotides and 3'-position-modified non-bridged nucleotides;
   - the 5'-side region comprises
      at least one nucleotide independently selected from the group consisting of deoxyribonucleotides, ribonucleotides and sugar moiety-modified nucleotides, and a 3'-terminal thereof being a sugar moiety-modified nucleotide, where the sugar moiety-modified nucleotide at the 3'-terminal binds to the central region, and is selected from the sugar moiety-modified nucleotides excluding a 2'-3' bridged nucleotide and 3'-position-modified non-bridged nucleotide, and
      does not contain an oligonucleotide strand constituted by at least four contiguous nucleotides which are independently selected from the group consisting of deoxyribonucleotides, 2'-3' bridged nucleotides and 3'-position-modified non-bridged nucleotides; and
   - the 3'-side region comprises
      at least one nucleotide independently selected from the group consisting of deoxyribonucleotides, ribonucleotides and sugar moiety-modified nucleotides, and a 5'-terminal thereof being a sugar moiety-modified nucleotide, where the sugar moiety-modified nucleotide at the 5'-terminal binds to the central region, and is selected from the sugar moiety-modified nucleotides excluding a 2'-3' bridged nucleotide and 3'-position-modified non-bridged nucleotide, and
      does not contain an oligonucleotide strand constituted by at least four contiguous nucleotides which are independently selected from the group consisting of deoxyribonucleotides, 2'-3' bridged nucleotides and 3'-position-modified non-bridged nucleotides.
2. The antisense oligonucleotide described in 1., wherein the central region comprises 5 to 15 nucleotides, and
   the 5'-side region and the 3'-side region each independently comprise 1 to 7 nucleotides.
3. The antisense oligonucleotide described in 1. or 2., wherein the central region comprises 8 to 12 nucleotides, and
   the 5'-side region and the 3'-side region each independently comprise 2 to 5 nucleotides.
4. The antisense oligonucleotide described in any one of 1. to 3., wherein 2'-3' bridged nucleotide contained in the central region is a nucleotide containing a partial structure represented by the following formula (I): 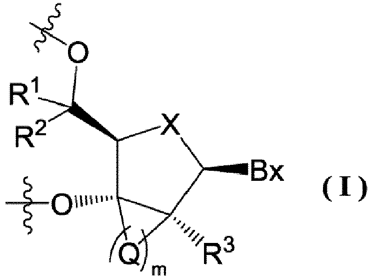
   (wherein m is 1, 2, 3 or 4,
   Bx is a nucleic acid base moiety,
   X is O or S,
   -Q-'s are each independently -CR⁴R⁵-, -C(=O)-, -C(=S)-, -C(=NR⁶)-, -O-, -NH-, -NR⁶- or -S-,
   when m is 2, 3 or 4, two adjacent -Q-'s may together form a group represented by the formula: -CR⁷=CR⁸-,
   R¹, R², R³, R⁴ and R⁵ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano, J¹, J² and J³ are each independently a hydrogen atom or C1-C6 alkyl, and Y is O, S or NJ⁴, J⁴ is C1-C12 alkyl or an amino protective group;
   R⁶ is C1-C12 alkyl or an amino protective group,
   R⁷ and R⁸ are each independently a hydrogen atom or C1-C6 alkyl).
5. The antisense oligonucleotide described in any one of 1. to 3., wherein 3'-position-modified non-bridged nucleotide contained in the central region is a nucleotide containing a partial structure represented by the following formula (II): 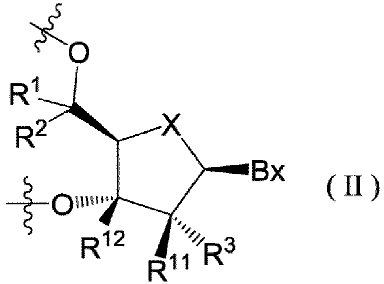
   (wherein Bx is a nucleic acid base moiety,
   X is O or S,
   R¹² is C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano; R¹, R², R³ and R¹¹ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano;
   J¹, J² and J³ are each independently a hydrogen atom or C1-C6 alkyl, and Y is O, S or NJ⁴, and J⁴ is C1-C12 alkyl or an amino protective group).
6. The antisense oligonucleotide described in 4., wherein the 2'-3' bridged nucleotide contained in the central region is a nucleotide represented by the following formula (III): 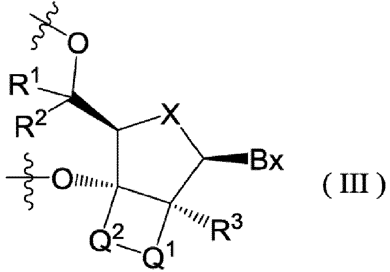
   (wherein Bx is a nucleic acid base moiety,
   X is O or S,
   -Q¹- and -Q²- are each independently -CR⁴R⁵-, -C(=O)-, -C(=S)-, -C(=NR⁶)-, -O-, -NH-, -NR⁶- or -S-, or,
   -Q¹-Q²- is -CR⁷=CR⁸-; and, wherein R⁷ and R⁸ are each independently a hydrogen atom or C1-C6 alkyl,
   R¹, R², R³, R⁴ and R⁵ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano, J¹, J² and J³ are each independently a hydrogen atom or C1-C6 alkyl, and Y is O, S or NJ⁴, J⁴ is C1-C12 alkyl or an amino protective group; and
   R⁶ is C1-C12 alkyl or an amino protective group).
7. The antisense oligonucleotide described in 6., wherein -Q¹- is -O-, -NH-, -NR⁶- or -S-, R⁶ is C1-C12 alkyl, and -Q²- is -CH₂-.
8. The antisense oligonucleotide described in 6. or 7., wherein -Q¹- is -O-, and -Q²- is -CH₂-.
9. The antisense oligonucleotide described in any one of 4. to 8., wherein R¹, R² and R³ are hydrogen atoms.
10. The antisense oligonucleotide described in any one of 4. to 9., wherein X is O.
11. The antisense oligonucleotide described in any one of 1. to 10., wherein the central region is a gap region,
   the 5'-side region is a 5'-wing region, and
   the 3'-side region is a 3'-wing region.
12. The antisense oligonucleotide described in any one of 1. to 11., wherein the sugar moiety-modified nucleotides contained in the 5'-side region and the 3'-side region are each independently selected from the group consisting of 2'-position-modified non-bridged nucleotide and 2',4'-BNA.
13. The antisense oligonucleotide described in 12., wherein the 2'-position-modified non-bridged nucleotide is at least one selected from the group consisting of 2'-O-methyl nucleotide, 2'-O-methoxyethyl (MOE) nucleotide, 2'-O-aminopropyl (AP) nucleotide, 2'-fluoronucleotide, 2'-O-(N-methylacetamido) (NMA) nucleotide and 2'-O-methylcarbamoylethyl (MCE) nucleotide .
14. The antisense oligonucleotide described in 12., wherein the 2',4'-BNA is at least one selected from the group consisting of LNA, cEt-BNA, ENA, BNA^{NC}, AmNA and scpBNA.
15. The antisense oligonucleotide described in any one of 1. to 14., wherein the antisense oligonucleotide contains a phosphorothioate bond.
16. The antisense oligonucleotide described in any one of 1. to 15., which further comprises a group derived from a functional molecule having at least one kind of a function selected from the group consisting of a labeling function, purifying function and delivering function to a target site.
17. The antisense oligonucleotide described in 16., wherein the functional molecule is selected from the group consisting of sugar, lipid, peptide and protein and their derivatives.
18. The antisense oligonucleotide described in 16. or 17., wherein the functional molecule is a lipid selected from the group consisting of cholesterol, tocopherol and tocotrienol.
19. The antisense oligonucleotide described in 16. or 17., wherein the functional molecule is a sugar derivative that interacts with an asialoglycoprotein receptor.
20. The antisense oligonucleotide described in 16. or 17., wherein the functional molecule is a peptide or protein selected from the group consisting of receptor ligands and antibodies.
21. A prodrug which comprises the antisense oligonucleotide described in any one of 1. to 20.
22. An oligonucleotide complex which comprises
   (i) the antisense oligonucleotide described in any one of 1. to 20., and
   (ii) an oligonucleotide containing at least one ribonucleotide, and containing a region that hybridizes with the (i) antisense oligonucleotide.
23. An oligonucleotide which comprises
   (i) a group derived from the antisense oligonucleotide described in any one of 1. to 20., and
   (ii) a group derived from an oligonucleotide containing at least one ribonucleotide, and containing a region that hybridizes with the antisense oligonucleotide of the above-mentioned (i), and
   the group derived from the antisense oligonucleotide of the above-mentioned (i), and the group derived from the oligonucleotide of the above-mentioned (ii) are linked.
24. An oligonucleotide complex which comprises
   (iii) an oligonucleotide in which an oligonucleotide strand containing at least one ribonucleotide is linked to the group derived from the antisense oligonucleotide described in any one of 1. to 20., and
   (iv) an oligonucleotide containing an oligonucleotide strand which contains at least four contiguous nucleotides recognized by RNase H, and
   the oligonucleotide strand containing at least one ribonucleotide of the above-mentioned (iii), and the oligonucleotide strand containing at least four contiguous nucleotides recognized by RNase H of the above-mentioned (iv) are hybridized.
25. An oligonucleotide which comprises
   (iii) a group derived from an oligonucleotide in which an oligonucleotide strand containing at least one ribonucleotide is linked to a group derived from the antisense oligonucleotide described in any one of 1. to 20., and
   (iv) a group derived from an oligonucleotide containing an oligonucleotide strand which contains at least four contiguous nucleotides recognized by RNase H, and the group derived from the oligonucleotide of the above-mentioned (iii) and the group derived from the oligonucleotide of the above-mentioned (iv) are linked, and
   the oligonucleotide strand containing at least one ribonucleotide of the above-mentioned (iii) and the oligonucleotide strand which contains at least four contiguous nucleotides recognized by RNase H of the above-mentioned (iv) are hybridized.
26. A pharmaceutical composition which comprises the antisense oligonucleotide described in any one of 1. to 20., the prodrug described in 21., the oligonucleotide complex described in 22. or 24., or the oligonucleotide described in 23. or 25., and a pharmacologically acceptable carrier.
27. A method for controlling a function of a target RNA, which comprises a step of contacting the antisense oligonucleotide described in any one of 1. to 20., the prodrug described in 21., the oligonucleotide complex described in 22. or 24., or the oligonucleotide described in 23. or 25., with a cell.
28. A method for controlling a function of a target RNA in a mammal, which comprises a step administering the pharmaceutical composition described in 26. to the mammal.
29. A method for controlling expression of a target gene, which comprises a step of contacting the antisense oligonucleotide described in any one of 1. to 20., the prodrug described in 21., the oligonucleotide complex described in 22. or 24., or the oligonucleotide described in 23. or 25., with a cell.
30. A method for controlling expression of a target gene in a mammal, which comprises a step of administering the pharmaceutical composition described in 26. to the mammal.
31. A method for producing the antisense oligonucleotide described in any one of 1. to 20., or the prodrug described in 21., which comprises using a nucleotide selected from the group consisting of 2'-3' bridged nucleotides and, 3'-position-modified non-bridged nucleotides.

### EFFECTS OF THE INVENTION

According to the present invention, an antisense oligonucleotide reduced in toxicity is provided.

### BRIEF DESCRIPTION OF THE DRAWINGS

FIG. 1 is a graph showing an effect of the antisense oligonucleotide (Example 1) according to the present embodiment on an expression level of SOD-1 in mouse brain endothelial cells.
FIG. 2 is a graph showing an effect of the antisense oligonucleotide (Example 1) according to the present embodiment on cell viability in mouse brain endothelial cells.
FIG. 3 is a graph showing an effect of the antisense oligonucleotide (Example 2) according to the present embodiment on cell viability in mouse brain endothelial cells.
FIG. 4 is a graph showing the results of a comprehensive analysis of the effect of the antisense oligonucleotide (Comparative Example 1) on changes in gene expression levels in mouse brain endothelial cells.
FIG. 5 is a graph showing the results of a comprehensive analysis of the effect of the antisense oligonucleotide (Example 1) according to the present embodiment on changes in gene expression levels in mouse brain endothelial cells.

### BEST MODE FOR CARRYING OUT THE INVENTION

The terms used in the present description are used in the sense in which they are ordinarily used in the art unless specifically indicated otherwise. The following provides an explanation of terms used in the present description. Furthermore, the terms used in the present description have the same meaning both in the case they are used alone and in the case they are used in conjunction with other terms unless otherwise specifically described.

"n-" refers to normal, "i-" iso, "s-" secondary, "t-" tertiary, "m-" meta, and "p-" para. "Ph" refers to phenyl, "Me" methyl, "Pr" propyl, "Bu" butyl, and "DMTr" dimethoxytrityl.

A functional group substituted by a protective group refers to a functional group in which a hydrogen atom possessed by the functional group is substituted by a protective group.

A "halogen atom" refers to a fluorine atom, a chlorine atom, a bromine atom or an iodine atom.

"C1-C12 alkyl" refers to a monovalent linear or branched saturated aliphatic hydrocarbon group having 1 to 12 carbon atoms. Examples of the C1-C12 alkyl include, for example, methyl, ethyl, n-propyl, isopropyl, n-butyl, isobutyl, s-butyl, t-butyl, n-pentyl, isopentyl, neopentyl, n-hexyl, n-heptyl, n-octyl, n-nonyl, n-decyl, n-undecyl, and n-dodecyl.

"C1-C6 alkyl" refers to a monovalent linear or branched saturated aliphatic hydrocarbon group having 1 to 6 carbon atoms among the above-mentioned "C1-C12 alkyl". Examples of the C1-C6 alkyl include, for example, methyl, ethyl, n-propyl, isopropyl, n-butyl, isobutyl, s-butyl, t-butyl, n-pentyl, isopentyl, neopentyl, n-hexyl, and isohexyl. Similarly, a "C1-C3 alkyl" refers to a monovalent linear or branched saturated aliphatic hydrocarbon group having 1 to 3 carbon atoms.

A "halo-C1-C6 alkyl" refers to a group in which at least one of hydrogen atoms at an optional position of the above-mentioned "C1-C6 alkyl" is substituted by the above-mentioned "halogen atom".

"C2-C6 alkenyl" refers to a monovalent linear or branched unsaturated aliphatic hydrocarbon having 2 to 6 carbon atoms containing at least one carbon-carbon double bond. Examples of the C2-C6 alkenyl include, for example, vinyl, allyl, propenyl, isopropenyl, butenyl, isobutenyl, butadienyl, 3-methyl-2-butenyl, pentenyl, isopentenyl, pentadienyl, hexenyl, isohexenyl, and hexadienyl.

"C2-C6 alkynyl" refers to a monovalent linear or branched unsaturated aliphatic hydrocarbon having 2 to 6 carbon atoms containing at least one carbon-carbon triple bond. Examples of the C2-C6 alkynyl include, for example, ethynyl, propargyl, 3-butynyl and 4-pentynyl.

"Acyl" refers to a group in which a hydrogen atom, C1-C6 alkyl, C2-C6 alkenyl or aryl is bound to a carbonyl (-C(=O)-) group. Examples of the acyl include, for example, formyl, acetyl, pivaloyl, and benzoyl.

"Haloacyl" refers to a group in which at least one of hydrogen atoms at an optional position of the above-mentioned "acyl" is substituted by the above-mentioned "a halogen atom".

"Amide" refers to an aminocarbonyl (-CONH₂) group, or a group in which at least one of hydrogen atoms of the aminocarbonyl group is substituted by a group independently selected from the group consisting of the C1-C6 alkyl, C2-C6 alkenyl and aryl. Examples of the amide include, for example, carbamoyl, methylaminocarbonyl, isopropylaminocarbonyl, and phenylaminocarbonyl.

"C1-C6 alkoxy" refers to a group in which the above-mentioned "C1-C6 alkyl" is bound to an oxy (-O-) group. Examples of the C1-C6 alkoxy include, for example, methoxy, ethoxy, n-propoxy, isopropoxy, n-butoxy, t-butoxy, isobutoxy, s-butoxy, n-pentyloxy, isopentyloxy, and n-hexyloxy.

"C1-C6 alkylthio" refers to a group in which the above-mentioned "C1-C6 alkyl" is bound to a thio (-S-) group. Examples of the C1-C6 alkylthio include, for example, methylthio, ethylthio, n-propylthio, isopropylthio, n-butylthio, isobutylthio, s-butylthio, t-butylthio, n-pentylthio, isopentylthio, and n-hexylthio.

"C2-C50 alkylene" refers to a divalent linear or branched saturated aliphatic hydrocarbon group having 2 to 50 carbon atoms.

"C2-C20 alkylene" refers to a divalent linear or branched saturated aliphatic hydrocarbon group having 2 to 20 carbon atoms.

"C8-C12 alkylene" refers to a divalent linear or branched saturated aliphatic hydrocarbon group having 8 to 12 carbon atoms among the above-mentioned "C2-C20 alkylene".

"C2-C6 alkylene" refers to a divalent linear or branched saturated aliphatic hydrocarbon group having 2 to 6 carbon atoms among the above-mentioned "C2-C20 alkylene", and examples thereof include ethylene (ethanediyl), propylene, propan-1,3-diyl (trimethylene), propan-2,2-diyl (isopropylidene), 2,2-dimethyl-propan-1,3-diyl, hexan-1,6-diyl (hexamethylene) and 3-methylbutan-1,2-diyl.

"C2-C20 alkenylene" refers to a divalent linear or branched unsaturated aliphatic hydrocarbon group having 2 to 20 carbon atoms containing at least one carbon-carbon double bond.

"Mono C1-C6 alkylamino" refers to a group in which at least one of hydrogen atoms of the amino (NH₂) group is substituted by the above-mentioned "C1-C6 alkyl" and examples thereof include, for example, methylamino, ethylamino, n-propylamino, isopropylamino, n-butylamino, isobutylamino, s-butylamino, t-butylamino, n-pentylamino, n-hexylamino and isohexylamino.

"Di C1-C6 alkylamino" refers to a group in which two hydrogen atoms of the amino (NH₂) group are substituted by the same or different two above-mentioned "C1-C6 alkyl"s and examples thereof include, for example, dimethylamino, diethylamino, di-n-propylamino, diisopropylamino, di-n-butylamino, di-n-pentylamino, di-n-hexylamino, N-methyl-N-ethylamino, and N-methyl-N-isopropylamino.

"C1-C6 alkylcarbonyl", "halo-C1-C6 alkylcarbonyl", "C1-C6 alkoxycarbonyl", "mono C1-C6 alkylaminocarbonyl" and "di C1-C6 alkylaminocarbonyl" each refer to a group in which the above-mentioned "C1-C6 alkyl", "halo-C1-C6 alkyl", "C1-C6 alkoxy", "mono C1-C6 alkylamino" and "di C1-C6 alkylamino" are bound to a carbonyl (-C(=O)-) group, respectively.

"C1-C6 alkylsulfonyl", "halo-C1-C6 alkylsulfonyl", "C1-C6 alkoxysulfonyl", "mono C1-C6 alkylaminosulfonyl" and "di C1-C6 alkylaminosulfonyl" each refer to a group in which the above-mentioned "C1-C6 alkyl", "halo-C1-C6 alkyl", "C1-C6 alkoxy", "mono C1-C6 alkylamino" and "di C1-C6 alkylamino" are bound to a sulfonyl group (-S(O)₂-), respectively.

A "ribonucleoside group" refers to a group in which a base is bound to a carbon atom at the 1'-position of a ribose, and hydroxy groups at the 3'-position and the 5'-position of the ribose are removed. The base moiety in the ribonucleoside group of the present invention may be a naturally-occurring base, or may be a base in which the naturally-occurring base is modified. The modification of the above-mentioned base moiety may be performed in combination of two or more kinds on one ribonucleoside group. The above-mentioned modifiication is described in, for example, Journal of Medicinal Chemistry (2016, vol. 59, No. 21, pp. 9645-9667), Medicinal Chemistry Communications (2014, vol. 5, pp. 1454-1471), and Future Medicinal Chemistry (2011, vol. 3, No. 3, pp. 339-365)

A "deoxyribonucleoside group" refers to a group in which a base is bound to a carbon atom at the 1'-position of 2'-deoxyribose, and hydroxy groups at the 3'-position and the 5'-position of the 2'-deoxyribose are removed. The base moiety in the deoxyribonucleoside group of the present invention may be a naturally-occurring base, or may be a base in which the naturally-occurring base is modified. The modification of the base moiety may be performed in combination of two or more kinds on one deoxyribonucleoside group. The above-mentioned modification is described in, for example, Journal of Medicinal Chemistry (2016, vol. 59, No. 21, pp. 9645-9667), Medicinal Chemistry Communications (2014, vol. 5, pp. 1454-1471), Future Medicinal Chemistry (2011, vol. 3, No. 3, pp. 339-365).

An "oxo" indicates a group a group (=O) in which the oxygen atom is substituted via a double bond. In the case an oxo is substituted for a carbon atom, it forms carbonyl together with the carbon atom.

A "thioxo" indicates a group (=S) in which the sulfur atom is substituted via a double bond. In the case a thioxo is substituted for a carbon atom, it forms thiocarbonyl together with the carbon atom.

A hydroxy protective group and an amino protective group are not particularly limited as long as they are stable when synthesizing an antisense oligonucleotide, and there may be mentioned protective groups well known to the persons of ordinary skill in the art, for example, as described in Protective Groups in Organic Synthesis, 4th edition, written by T. W. Greene, P. G. M. Wuts, John Wiley & Sons Inc. (2006). For example, as the "amino protective group", there may be mentioned amide-based protective groups such as acyl (for example, formyl, acetyl, propionyl, pivaloyl (Pv), and tigloyl may be mentioned), haloacyl (for example, fluoroacetyl, difluoroacetyl, trifluoroacetyl, chloroacetyl, dichloroacetyl, and trichloroacetyl may be mentioned), and arylcarbonyl (for example, benzoyl, p-bromobenzoyl, p-nitrobenzoyl, and 2,4-dinitrobenzoyl may be mentioned); carbamate-based protective groups such as C1-C6 alkoxycarbonyl (for example, methoxycarbonyl, ethoxycarbonyl, n-propoxycarbonyl, i-propoxycarbonyl, n-butoxycarbonyl, i-butoxycarbonyl, t-butoxycarbonyl (Boc), and t-amyloxycarbonyl may be mentioned, and preferably Boc may be mentioned), C2-C6 alkenyloxycarbonyl (for example, vinyloxycarbonyl (Voc), and allyloxycarbonyl (Alloc) may be mentioned), tri(C1-C3 alkyl)silylethoxycarbonyl (for example, 2-(trimethylsilyl)ethoxycarbonyl (Teoc) may be mentioned), halo-C1-C6 alkoxycarbonyl (for example, 2,2,2-trichloroethoxycarbonyl (Troc) may be mentioned), and aryloxycarbonyl (for example, benzyloxycarbonyl (Z or Cbz), p-nitrobenzyloxycarbonyl, and p-methoxybenzyloxycarbonyl (Moz) may be mentioned); and sulfonamide-based protective groups such as alkylsulfonyl (for example, methanesulfonyl (Ms), and ethanesulfonyl may be mentioned), and arylsulfonyl (for example, benzenesulfonyl, p-toluenesulfonyl (Ts), p-chlorobenzenesulfonyl, p-methoxybenzenesulfonyl (MBS), m-nitrobenzenesulfonyl, o-nitrobenzenesulfonyl, p-nitrobenzenesulfonyl, 2,4-nitrobenzenesulfonyl, 2,6-dimethoxy-4-methylbenzenesulfonyl (iMds), 2,6-dimethyl-4-methoxybenzenesulfonyl (Mds), 2,4,6-trimethoxybenzenesulfonyl (Mtb), 2,3,5,6-tetramethyl-4-methoxybenzenesulfonyl (Mte), 2,3,6-trimethyl-4-methoxybenzenesulfonyl (Mtr), 2,4,6-trimethylbenzenesulfonyl (Mts), and pentamethylbenzenesulfonyl (Pme) may be mentioned).

With regard to protection and deprotection of the "hydroxy protective group" and the "amino protective group" in the present invention, it is possible to refer to Protective Groups in Organic Synthesis, 4th Edition, written by T. W. Greene, P. G. M. Wuts, John Wiley & Sons Inc. (2006).

"Antisense effect" refers to controlling the function of a target RNA by hybridizing a target RNA selected corresponding to a target gene and, for example, an oligonucleotide having a sequence complementary to a partial sequence thereof. For example, in the case the target RNA is mRNA, an antisense effect refers to translation of the above-mentioned target RNA being inhibited by hybridization, an effect that converts a splicing function such as exon skipping, or the above-mentioned target RNA being degraded as a result of recognition of a hybridized portion.

An "antisense oligonucleotide" is an oligonucleotide that produces the above-mentioned antisense effect. For example, there may be mentioned DNA and oligodeoxyribonucleotides, but are not limited thereto, and may be RNA, oligoribonucleotides, or oligonucleotides designed to normally produce the antisense effect. The same applies to antisense nucleic acids.

"Target RNA" refers to mRNA, mRNA precursor or ncRNA, and includes mRNA transcribed from genomic DNA encoding a target gene, mRNA not subjected to base modification, and mRNA precursor or ncRNA that have not been subjected to splicing. There are no particular limitations on the "target RNA" for which the function thereof is controlled by an antisense effect, and examples thereof include RNA associated with genes for which expression increases in various diseases. The "target RNA" may be any RNA synthesized by DNA-dependent RNA polymerase, and is preferably mRNA or mRNA precursor. It is more preferably mammal mRNA or mRNA precursor, more preferably human mRNA or mRNA precursor, and particularly preferably human mRNA.

"Hybridize" refers to the act of forming a double-strand between oligonucleotides containing complementary sequences or groups derived from those oligonucleotides, and constitutes a phenomenon in which oligonucleotides containing complementary sequences or groups derived from those oligonucleotides form a double strand.

"Complementary" refers to two nucleic acid bases being able to form a Watson-Crick base pair (naturally-occurring base pair) or non-Watson-Crick base pair (such as a Hoogsteen base pair) via hydrogen bonds. Two oligonucleotides or groups derived from those oligonucleotides are able to "hybridize" in the case their sequences are complementary. Although it is not necessary for sequences to be completely complementary in order for two oligonucleotides or groups derived from those oligonucleotides to hybridize, complementarity for two oligonucleotides or groups derived from those oligonucleotides to hybridize is preferably 70% or more, more preferably 80% or more and even more preferably 90% or more (such as 95%, 96%, 97%, 98% or 99% or more). Sequence complementarity can be determined by using a computer program that automatically identifies the partial sequences of oligonucleotides. One example of software used for that purpose is, for example, OligoAnalyzer available from Integrated DNA Technologies. This program can also be accessed online from a Web site. The persons of ordinary skill in the art is therefore able to easily determine conditions (such as temperature or salt concentration) for enabling hybridization of two oligonucleotides or groups derived from those oligonucleotides. In addition, the persons of ordinary skill in the art can easily design an antisense oligonucleotide complementary to target RNA by, for example, using software such as the BLAST program based on information of the nucleotide sequence data of the target RNA. With respect to the BLAST program, literature such as Proceedings of the National Academy of Science of the United States of America, 1990, 87, pp. 2264-2268; Ditto 1993, 90, pp. 5873-5877, and the Journal of Molecular Biology, 1990, 215, pp. 403-410 can be referred to.

A "nucleotide" refers to a molecule capable of serving as a structural unit of a nucleic acid (oligonucleotide), and normally has a base as constituents thereof. A nucleotide is composed of, for example, a sugar, a base and a phosphoric acid. Nucleotides include ribonucleotides, deoxyribonucleotides and sugar moiety-modified nucleotides mentioned later.

An "oligonucleotide" refers to a molecule having a structure in which one or more above-mentioned nucleotides are polymerized. When the "oligonucleotide" is composed of one nucleotide, that oligonucleotide can also be referred to as a "nucleotide".

Nucleotides contained in the "antisense oligonucleotide" molecule of the present invention are each independently coupled to each other by a phosphodiester bond, a modified phosphodiester bond mentioned later or a linking group that contains a non-nucleotide structure mentioned later. The nucleotide at the 3'-end of the antisense oligonucleotide molecule of the present invention preferably has a hydroxyl group or a phosphate group at the 3'-position, more preferably has a hydroxyl group, and usually has a hydroxyl group. The nucleotide at the 5'-end of the antisense oligonucleotide molecule preferably has a hydroxyl group or a phosphate group at the 5'-position, more preferably has a hydroxyl group, and usually has a hydroxyl group.

A "group derived from an oligonucleotide" refers to the partial structure of an oligonucleotide formed by removing a hydrogen atom or hydroxyl group and the like from at least one of the hydroxyl groups on the 3'-end or 5'-end of the above-mentioned oligonucleotide, and coupled with the other group (for example, other groups derived from an oligonucleotide) by forming a phosphodiester bond or a modified phosphodiester bond indirectly through a covalent bond. The above-mentioned hydroxyl group at the 3'-end or 5'-end include a hydroxyl group possessed by a phosphate group. For example, a group in which a hydrogen atom is removed from the hydroxyl group at the 3'-end of the oligonucleotide and a group in which a hydroxyl group is removed from the phosphate group at the 5'-end of the oligonucleotide forms a phosphodiester bond or a modified phosphodiester bond.

A "nucleotide sequence" refers to the base sequence of nucleotides that compose an oligonucleotide.

In the present description, a "sequence portion" refers to a partial structure of an oligonucleotide strand. For example, a sequence portion containing nucleotides is a partial structure of a region of an oligonucleotide strand that contains the nucleotides.

A "deoxyribonucleotide" refers to a molecule in which the sugar is 2'-deoxyribose, a base is bound to a carbon atom at the 1'-position of 2'-deoxyribose, and a phosphate group is bound to the 3'-position or 5'-position. The deoxyribonucleotide in the present invention may be a naturally-occurring deoxyribonucleotide or a deoxyribonucleotide in which the base moiety or phosphodiester bond portion of the naturally-occurring deoxyribonucleotide is modified. Modification of the base moiety and modification of the phosphodiester bond portion may be performed on combination of a plurality of types modification on one deoxyribonucleotide. The above-mentioned modified deoxyribonucleotide is described in, for example, Journal of Medicinal Chemistry, 2016, vol. 59, pp. 9645-9667, Medicinal Chemistry Communication, 2014, vol. 5, pp. 1454-1471, and Future Medicinal Chemistry, 2011, vol. 3, pp. 339-365.

When the above-mentioned "deoxyribonucleotide" composes the antisense oligonucleotide molecule of the present invention, normally the 3'-position of the deoxyribonucleotide is coupled to another nucleotide through a phosphodiester bond or a modified phosphodiester bond (for example, a phosphorothioate bond), and the 5'-position of the deoxyribonucleotide is coupled to another nucleotide through a phosphodiester bond or a modified phosphodiester bond (for example, a phosphorothioate bond). The deoxyribonucleotide at the 3'-end of the antisense oligonucleotide molecule of the present invention preferably has a hydroxyl group or a phosphate group at the 3'-position, and the 5'-position is as previously described. The deoxyribonucleotide at the 5'-end of the antisense oligonucleotide molecule preferably has a hydroxyl group or a phosphate group at the 5'-position, and the 3'-position is as previously described.

An "oligodeoxyribonucleotide" refers to an oligonucleotide that is composed of the above-mentioned deoxyribonucleotides. Deoxyribonucleotides composing the oligodeoxyribonucleotide may each be the same or different.

"DNA" refers to an oligonucleotide that is composed of naturally-occurring deoxyribonucleotides. The naturally-occurring deoxyribonucleotides that compose the DNA may each be the same or different.

A "ribonucleotide" refers to a molecule in which a sugar is ribose, a base is bound to a carbon atom at the 1'-position of the ribose and a phosphate group is present at the 2'-position, 3'-position or 5'-position. The ribonucleotide in the present invention may be a naturally-occurring ribonucleotide or may be a ribonucleotide in which a base moiety or a phosphodiester bond portion of the naturally-occurring ribonucleotide has been modified. Modification of the base moiety and modification of the phosphodiester bond portion may be performed on a combination of a plurality of types of modifications on a one ribonucleotide. The above-mentioned modified ribonucleotide is described in, for example, Journal of Medicinal Chemistry, 2016, vol. 59, pp. 9645-9667, Medicinal Chemistry Communication, 2014, vol. 5, pp. 1454-1471, and Future Medicinal Chemistry, 2011, vol. 3, pp. 339-365.

When the above-mentioned "ribonucleotide" composes an antisense oligonucleotide molecule of the present invention, typically the 3'-position of the ribonucleotide is coupled to another nucleotide through a phosphodiester bond or a modified phosphodiester bond (for example, a phosphorothioate bond), and the 5'-position of the ribonucleotide is coupled to another nucleotide through a phosphodiester bond or a modified phosphodiester bond (for example, a phosphorothioate bond). The ribonucleotide at the 3'-end of the antisense oligonucleotide molecule of the present invention preferably has a hydroxyl group or a phosphate group at the 3'-position thereof, and the 5'-position is as previously described. The ribonucleotide at the 5'-end of the antisense oligonucleotide molecule preferably has a hydroxyl group or a phosphate group at the 5'-position thereof, and the 3'-position is as previously described.

An "oligoribonucleotide" refers to an oligonucleotide that is composed of the above-mentioned ribonucleotide. The ribonucleotide that compose the oligoribonucleotide may each be the same or different.

"RNA" refers to an oligonucleotide that is composed of naturally-occurring ribonucleotides. The naturally-occurring ribonucleotides that compose the RNA may each be the same or different.

"Sugar moiety-modified nucleotide" refers to a nucleotide in which the sugar moiety of the above-mentioned deoxyribonucleotide or ribonucleotide is partially substituted with one or more substituents, the entire sugar backbone thereof has been replaced with a sugar backbone differing from ribose and 2'-deoxyribose (for example, a 5- or 6-membered sugar backbone such as hexitol and threose), the entire sugar backbone thereof or a portion of the ring of the sugar backbone has been replaced with a 5- to 7-membered saturated or unsaturated ring (for example, cyclohexane, cyclohexene, morpholine, and the like) or with a partial structure (for example, peptide structure) that allows the formation of a 5- to 7-membered ring by hydrogen bonding, or the ring of the sugar moiety is ring-opened, or further, the ring-opened portion is modified. A base moiety of a "sugar moiety-modified nucleotide" may be a naturally-occurring base or a modified base. In addition, a phosphodiester bond moiety of a "sugar moiety-modified nucleotide" may be a phosphodiester bond or a modified phosphodiester bond. Modification of a base moiety or modification of a phosphodiester bond portion on a single sugar moiety-modified nucleotide may be carried out on a combination of a plurality of types of modifications. Modification of the above-mentioned ring-opened portion may include, for example, halogenation, alkylation (for example, methylation, and ethylation), hydroxylation, amination, and thionation as well as demethylation.

A "sugar moiety-modified nucleotide" may be a bridged nucleotide or non-bridged nucleotide. Examples of sugar moiety-modified nucleotides include nucleotides disclosed as being preferable for use in an antisense method in, for example, Japanese Unexamined Patent Publication No. H10-304889, International Publication No. WO 2005/021570, Japanese Unexamined Patent Publication No. H10-195098, Japanese Translation of PCT Application No. 2002-521310, International Publication No. WO 2007/143315, International Publication No. WO 2008/043753, International Publication No. WO 2008/029619, Journal of Medicinal Chemistry, 2008, vol. 51, p 2766 or International Publication No. 2008/049085 (these documents are to be collectively referred to as "antisense method-related documents"). The above-mentioned documents disclose nucleotides such as hexitol nucleotides (HNA), cyclohexene nucleotides (CeNA), peptide nucleic acids (PNA), glycol nucleic acids (GNA), threose nucleotides (TNA), morpholino nucleic acids, tricyclo-DNA (tcDNA), 2'-O-methyl nucleotides, 2'-O-methoxyethyl (MOE) nucleotides, 2'-O-aminopropyl (AP) nucleotides, 2'-fluoronucleotides, 2'-F-arabinonucleotides (2'-F-ANA), bridged nucleotides (BNA (Bridged Nucleic Acid)), 2'-O-(N-methylacetamido)(NMA) nucleotide, and 2'-O-methylcarbamoylethyl (MCE) nucleotides. Further, Bioorganic & Medicinal Chemistry Letters, 2008, 18, pp. 2296-2300 (the above-mentioned Non-Patent Document 1), The Journal of Biological Chemistry, 2004, 279, pp. 36317-36326(the above-mentioned Non-Patent Document 2) disclose nucleotides such as 2'-3' bridged nucleotides and 3'-position-modified non-bridged nucleotides. In addition, sugar moiety-modified nucleotides are also disclosed in the literature such as Journal of Medicinal Chemistry, 2016, vol. 59, pp. 9645-9667, Medicinal Chemistry Communication, 2014, vol. 5, 1454-1471, and Future Medicinal Chemistry, 2011, vol. 3, pp. 339-365.

When the above-mentioned "sugar moiety-modified nucleotide" composes the antisense oligonucleotide molecule of the present invention, for example, the 3'-position of the sugar moiety-modified nucleotide is coupled to another nucleotide through a phosphodiester bond or modified phosphodiester bond (for example, a phosphorothioate bond), and the 5'-position of the sugar moiety-modified nucleotide is coupled to another nucleotide through a phosphodiester bond or modified phosphodiester bond (for example, a phosphorothioate bond). A sugar moiety-modified nucleotide on the 3'-end of the antisense oligonucleotide molecule of the present invention preferably has, for example, a hydroxyl group or phosphate group at the 3'-position thereof, and the 5'-position is as previously described. A sugar moiety-modified nucleotide on the 5'-end of the antisense oligonucleotide preferably has, for example, a hydroxyl group or phosphate group at the 5'-positon thereof and the 3'-position is as previously described.

Examples of modification of a phosphodiester bond moiety in deoxyribonucleotides, ribonucleotides and sugar moiety-modified nucleotides include phosphorothioation, methylphosphonation (including chiral-methylphosphonation), methylthiophosphonation, phosphorodithioation, phosphoroamidation, phosphorodiamidation, phosphoroamidothioation and boranophosphorylation. In addition, examples of the modification of the phosphodiester bond moiety in nucleotides are described in, for example, Journal of Medicinal Chemistry, 2016, vol. 59, pp. 9645-9667, Medicinal Chemistry Communications, 2014, vol. 5, pp. 1454-1471 and Future Medicinal Chemistry, 2011, vol. 3, pp. 339-365, and these can be used at the phosphodiester bond moiety in deoxyribonucleotides, ribonucleotides and sugar moiety-modified nucleotides.

A "bridged nucleotide" refers to a sugar moiety-modified nucleotide in which a bridging unit has been substituted by substitutions at two locations in a sugar moiety, and an example thereof includes 2'-4' bridged nucleotide, and 2'-3' bridged nucleotide and 3'-5' bridged nucleotide.

The 2'-4' bridged nucleotide (2',4'-BNA) is a nucleotide having a sugar moiety in which a carbon atom at the 2'-position and a carbon atom at the 4'-position are bridged by two or more atoms and may be mentioned, for example, a nucleotide having a sugar moiety bridged by C2-C6 alkylene (the alkylene is unsubstituted, or substituted by one or more substituents selected from the group consisting of a halogen atom, oxo and thioxo, and 1 or 2 methylene(s) of the alkylene is/are not replaced, or independently replaced with a group selected from the group consisting of -O-, -NR¹³-(R¹³ represents a hydrogen atom, C1-C6 alkyl or halo-C1-C6 alkyl) and -S-).

By combining the above-mentioned substitution and replacement, the group which bridges the 2'-position and the 4'-position of 2',4'-BNA may contain a group represented by -C(=O)-O-, -O-C(=O)-NR¹³- (R¹³ represents a hydrogen atom, C1-C6 alkyl or halo-C1-C6 alkyl), -C(=O)-NR¹³- (R¹³ represents a hydrogen atom, C1-C6 alkyl or halo-C1-C6 alkyl), or -C(=S)-NR¹³- (R¹³ represents a hydrogen atom, C1-C6 alkyl or halo-C1-C6 alkyl).

As such a BNA, there may be mentioned, for example, locked nucleic acid (Locked Nucleic Acid (Registered Trademark)) also referred to as LNA, α-L-methyleneoxy (4'-CH₂-O-2') BNA or β-D-methyleneoxy (4'-CH₂-O-2') BNA, ethyleneoxy (4'-(CH₂)₂-O-2') BNA also referred to as ENA, β**-**D-thio(4'-CH₂-S-2')BNA, aminooxy (4'-CH₂-O-N(R²¹)-2') BNA (R²¹ is H or CH₃), oxyamino (4'-CH₂-N(R²²)-O-2') BNA (R²² is H or CH₃) also referred to as 2',4'-BNA^{NC}, 2',4'-BNA^{COC}, 3'-amino-2',4'-BNA, 5'-methylBNA, (4'-CH(CH₃)-O-2') BNA also referred to as cEt-BNA, (4'-CH(CH₂OCH₃)-O-2') BNA also referred to as cMOE-BNA, amide type BNA (4'-C(=O)-N(R¹⁴)-2') BNA (R¹⁴ is H or CH₃) also referred to as AmNA, (4'-C(spiro-cyclopropyl)-O-2') BNA also referred to as scpBNA, and other BNA known for the persons of ordinary skill in the art.

The 2'-3' bridged nucleotide is a nucleotide having a sugar moiety in which a carbon atom at the 2'-position and a carbon atom at the 3'-position are bridged by one or more atoms and may be mentioned, for example, a nucleotide having a partial structure (sugar moiety and base moiety) represented by the following formula (I).

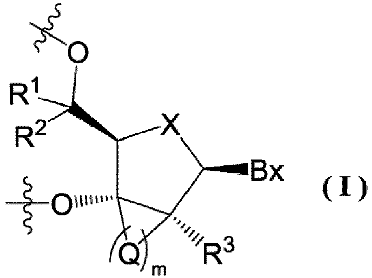

In the formula, m is 1, 2, 3 or 4,
Bx is a nucleic acid base moiety,
X is O or S,
-Q-'s are each independently -CR⁴R⁵-, -C(=O)-, -C(=S)-, -C(=NR⁶)-, -O-, -NH-, -NR⁶- or -S-,
when m is 2, 3 or 4, two adjacent -Q-'s may together form a group represented by the formula: -CR⁷=CR⁸-,
R¹, R², R³, R⁴ and R⁵ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano, J¹, J² and J³ are each independently a hydrogen atom or C1-C6 alkyl, Y is O, S or NJ⁴, and J⁴ is C1-C12 alkyl or an amino protective group;
R⁶ is C1-C12 alkyl or an amino protective group, and
R⁷ and R⁸ are each independently a hydrogen atom or C1-C6 alkyl.

The 3'-5' bridged nucleotide is a nucleotide having a sugar moiety in which a carbon atom at the 3'-position and a carbon atom at the 5'-position are bridged by two or more atoms. It may be mentioned, for example, tricyclo-DNA(tcDNA).

The 3'-position-modified non-bridged nucleotide is a non-bridged nucleotide in which a carbon atom at the 3'-position is modified and may be mentioned, for example, a nucleotide having a partial structure (sugar moiety and base moiety) represented by the following formula (II).

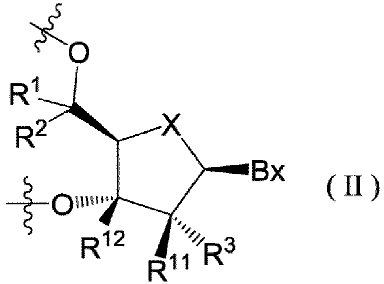

In the formula, Bx is a nucleic acid base moiety,
X is O or S,
R¹² is C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano; R¹, R², R³ and R¹¹ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano;
J¹, J² and J³ are each independently a hydrogen atom or C1-C6 alkyl, Y is O, S or NJ⁴, and J⁴ is C1-C12 alkyl or an amino protective group.

The 2'-position-modified non-bridged nucleotide is a non-bridged nucleotide in which an oxygen atom or a carbon atom at the 2'-position is modified and may be mentioned, for example, 2'-O-methyl nucleotide, 2'-O-methoxyethyl (MOE) nucleotide, 2'-O-aminopropyl (AP) nucleotide, 2'-fluoronucleotide, 2'-O-(N-methylacetamido) (NMA) nucleotide and 2'-O-methylcarbamoylethyl (MCE) nucleotide.

The sugar moiety-modified nucleotide is not necessarily limited to that exemplified here. A large number of the sugar moiety-modified nucleotides are known in this field of the art and sugar moiety-modified nucleotides described in, for example, U.S. Patent No. 8,299,039 to Tachas, et al. (in particular, columns 17 to 22), or Journal of Medicinal Chemistry, 2016, vol. 59, pp. 9645-9667, Medicinal Chemistry Communication, 2014, vol. 5, pp. 1454-1471, and Future Medicinal Chemistry, 2011, vol. 3, pp. 339-365 can be also used as embodiments of the present invention.

The persons of ordinary skill in the art are able to suitably select and use a sugar moiety-modified nucleotide from among such sugar moiety-modified nucleotides in consideration of viewpoints such as an antisense effect, affinity for a partial sequence of a target RNA and resistance to nuclease.

The "nucleic acid base" generally refers to a base component constituting the nucleic acid, and as a naturally-occurring nucleic acid base, purine bases such as adenine (A) and guanine (G), and pyrimidine bases such as thymine (T), cytosine (C) and uracil (U) are contained. In the base moiety of the deoxyribonucleotide, ribonucleotide and sugar moiety-modified nucleotide used in the present description, a naturally-occurring nucleic acid base and its modified nucleic acid base can be used. The modified nucleic acid base can form a base pair (that is, capable of forming a hydrogen bond) with any nucleic acid base (preferably a base complementary to the nucleic acid base before modification). Typically, the modified nucleic acid bases include 5-methylcytosine (5-me-C), 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halo uracil and cytosine, 5-propynyl (-C≡C-CH₃) of pyrimidine bases such as uracil and cytosine and other alkynyl derivatives, 6-azo uracil, cytosine and thymine, 5-uracil (pseudouracil), 4-thiouracil, 8-halo, 8-amino, 8-thiol, 8-thioalkyl, 8-hydroxyl and other 8-position substituted adenine and guanine, 5-halo, in particular, 5-bromo, 5-trifluoromethyl and other 5-position substituted uracil, and cytosine, 7-methylguanine and 7-methyladenine, 2-F-adenine, 2-amino-adenine, 8-azaguanine and 8-azaadenine, 7-deazaguanine and 7-deazaadenine, 3-deazaguanine and 3-deazaadenine. The further modified nucleic acid bases include tricyclic-based pyrimidines such as phenoxazine cytidine (1H-pyrimido[5,4-b][1,4]benzoxazin-2(3H)-one), phenothiazine cytidine (1H-pyrimido[5,4-b][1,4]benzothiazin-2(3H)-one), G-clamp such as substituted phenoxazine cytidine (for example, 9-(2-aminoethoxy)-H-pyrimid[5,4-b][1,4]benzoxazin-2(3H)-one), carbazole cytidine (2H-pyrimid[4,5-b]indol-2-one), and pyridoindole cytidine (H-pyrido[3',2':4,5]pyrrolo[2,3-d]pyrimidin-2-one). Also, the modified nucleic acid bases may contain a material in which purine or a pyrimidine base is substituted by another heterocycle, for example, 7-deazaadenine, 7-deazaguanosine, 2-aminopyridine and 2-pyridone. In addition, examples of the modification of the base moiety in the nucleotide are disclosed in Journal of Medicinal Chemistry, 2016, vol. 59, pp. 9645-9667, Medicinal Chemistry Communication, 2014, vol. 5, pp. 1454-1471, Future Medicinal Chemistry, 2011, vol. 3, pp. 339-365, and WO 2007/090071, and these can be used for the base moiety in the deoxyribonucleotide, ribonucleotide and sugar moiety-modified nucleotide. The amino and hydroxy of the base moiety may each independently protected.

The base moiety in the deoxyribonucleotide, ribonucleotide and sugar moiety-modified nucleotide is preferably at least one kind selected from the group consisting of adenine (A), guanine (G), thymine (T), cytosine (C), uracil (U) and 5-methylcytosine (5-me-C).

"RNase H" is generally known to be a ribonuclease which recognizes a double strand obtained by hybridizing DNA and RNA, and cleaves the RNA to generate a single-stranded DNA. RNase H is able to recognize not limited only to a double strand obtained by hybridizing DNA and RNA, but also to a double strand in which at least one of the base moiety, phosphodiester bond moiety and sugar moiety of at least one of DNA and RNA. For example, it is able to recognize a double strand in which an oligodeoxyribonucleotide and an oligoribonucleotide are hybridized.

Accordingly, DNA can be recognized by RNase H when hybridizing with RNA. This applies similarly in the case where at least one of a base moiety, phosphodiester bond moiety and sugar moiety has been modified in at least one of DNA and RNA. For example, a representative example thereof may be mentioned an oligonucleotide in which a phosphodiester bond moiety of DNA is modified to phosphorothioate.

RNA can be cleaved by RNase H when it is hybridized with DNA. This applies similarly in the case at least one of the base moiety, phosphodiester bond moiety and sugar moiety has been modified in at least one of DNA and RNA.

Examples of modifying of DNA and/or RNA able to be recognized by RNase H are described in, for example, Nucleic Acids Research, 2014, vol. 42, pp. 5378-5389, Bioorganic & Medicinal Chemistry Letters, 2008, vol. 18, pp. 2296-2300 (the above-mentioned Non-Patent Document 1), Molecular BioSystems, 2009, vol. 5, pp. 838-843, Nucleic Acid Therapeutics, 2015, vol. 25, pp. 266-274, The Journal of Biological Chemistry, 2004, vol. 279, pp. 36317-36326 (the above-mentioned Non-Patent Document 2).

The RNase H used in the present invention is preferably mammal RNase H, more preferably human RNase H, and particularly preferably human RNase HI.

The "gap region" is a region containing "at least four contiguous nucleotides recognized by RNase H" and is not particularly limited as long as it contains four or more contiguous nucleotides, and recognized by RNase H, and the contiguous nucleotides are preferably independently selected from deoxyribonucleotides and sugar moiety-modified nucleotides.

The "5'-wing region" is a region linked to the 5'-side of the gap region and contains "at least one nucleotide" without containing the above-mentioned "at least four contiguous nucleotides recognized by RNase H", where the sugar moiety of the nucleotide at the 3'-end of 5'-wing region is different from the sugar moiety of the nucleotide at the 5'-end of the gap region. Due to the difference of the sugar moiety, the boundary between the 5'-wing region and the gap region can be confirmed. (for example, the nucleotide at the 5'-end of the gap region is a deoxyribonucleotide, and the nucleotide at the 3'-end of the 5'-wing region is a sugar moiety-modified nucleotide.) The nucleotide at the 3'-end of the 5'-wing region is generally a sugar moiety-modified nucleotide. The 5'-wing region is not particularly limited as long as it satisfies the above definition, and the at least one nucleotide is preferably independently selected from deoxyribonucleotides and sugar moiety-modified nucleotides, and contains at least one sugar moiety-modified nucleotide.

The "3'-wing region" is a region linked to the 3'-side of the gap region and contains "at least one nucleotide" without containing the above-mentioned "at least four contiguous nucleotides recognized by RNase H", where sugar moiety of the nucleotide at the 5'-end of the 3'-wing region is different from the sugar moiety of the nucleotide at the 3'-end of the gap region. Due to the difference of the sugar moiety, the boundary between the 3'-wing region and the gap region can be confirmed. (for example, the nucleotide at the 3'-end of the gap region is a deoxyribonucleotide, and the nucleotide at the 5'-end of the 3'-wing region is a sugar moiety-modified nucleotide.) The nucleotide at the 5'-end of the 3'-wing region is generally a sugar moiety-modified nucleotide. The 3'-wing region is not particularly limited as long as it satisfies the above definition, and the at least one nucleotide is preferably independently selected from deoxyribonucleotides and sugar moiety-modified nucleotides, and contains at least one sugar moiety-modified nucleotide.

An antisense oligonucleotide having a gap region, a 5'-wing region and a 3'-wing region is called a gapmer type antisense oligonucleotide.

The "the central region" is a central region in the oligonucleotide.

The "5'-side region" is a region linked to the 5'-side of the above-mentioned "the central region", and contains at least one nucleotide.

The "3'-side region" is a region linked to the 3'-side of the above-mentioned "the central region", and contains at least one nucleotide.

The sugar moiety of the nucleotide at the 5'-end of the 3'-side region is different from the sugar moiety of the nucleotide at the 3'-end of the central region. Due to the difference of the sugar moiety, the boundary of the 3'-side region and the central region can be confirmed. The sugar moiety of the nucleotide at the 3'-end of the 5'-side region is different from the sugar moiety of the nucleotide at the 5'-end of the central region. Due to the difference of the sugar moiety, the boundary of the 5'-side region and the central region can be confirmed.

The "at least four contiguous nucleotides recognized by RNase H" is not particularly limited as long as it contains four or more contiguous nucleotides and can be recognized by RNase H, and may be mentioned, for example, "at least four contiguous deoxyribonucleotides" and "at least four contiguous nucleotides which are independently selected from the group consisting of the deoxyribonucleotide, 2'-3' bridged nucleotide and 3'-position modified non-bridged nucleotide". A number of the nucleotides is, for example, 5 to 30, preferably 5 to 15, and more preferably 8 to 12.

The persons of ordinary skill in the art can judge whether a certain at least four contiguous nucleotides are "at least four contiguous nucleotides recognized by RNase H" or not by the structure of the sugar moiety of the contiguous nucleotides.

Next, the antisense oligonucleotide of the present invention is explained.

The antisense oligonucleotide of the present invention does not necessarily hybridize with the entire target RNA, and may hybridize with at least a part of the target RNA, and usually hybridizes with at least a part of the target RNA. For example, by hybridizing an oligonucleotide (DNA, an oligodeoxyribonucleotide or an oligonucleotide designed to usually produce an antisense effect) having an antisense sequence complementary to a partial sequence of the target RNA with at least a part of the target RNA, expression of the target gene is controlled. Also, the entire part of the antisense oligonucleotide is not necessarily hybridized, and a part thereof may not hybridize. The entire part of the antisense sequence portion may not hybridize at a part thereof, but preferably hybridize.

Incidentally, the "antisense sequence" refers to a base sequence of nucleotides that constitute an oligonucleotide that enables hybridization with the target RNA, and the "antisense sequence portion" refers to a partial structure at the region having the above-mentioned antisense sequence in the oligonucleotide strand.

The complementarity between the antisense sequence portion of the above-mentioned antisense oligonucleotide and the partial sequence of the target RNA is preferably 70% or more, more preferably 80% or more, further preferably 90% or more (for example, 95%, 96%, 97%, 98% or 99% or more). Although it is not necessary for these sequences to be completely complementary in order for hybridizing the antisense sequence portion of the antisense oligonucleotide with at least a part of the target RNA, it is further more preferably completely complementary.

The persons of ordinary skill in the art can easily determine the base sequence compatible with the antisense sequence "enabling hybridization with the target RNA" by using the BLAST program or the like.

The antisense oligonucleotide of the present invention has a central region, a 5'-side region and a 3'-side region. The central region is preferably a gap region, the 5'-side region is preferably a 5'-wing region, and the 3'-side region is a 3'-wing region.

The central region comprises at least 5 nucleotides independently selected from the group consisting of deoxyribonucleotides, ribonucleotides and sugar moiety-modified nucleotides, and contains at least one sugar moiety-modified nucleotide selected from the group consisting of 2'-3' bridged nucleotides and 3'-position modified non-bridged nucleotides, the 3'-end and the 5'-end thereof are each independently a deoxyribonucleotide, ribonucleotide, 2'-3' bridged nucleotide or 3'-position modified non-bridged nucleotide, and contains at least one oligonucleotide strand constituted by at least four contiguous nucleotides which are independently selected from the group consisting of deoxyribonucleotides, 2'-3' bridged nucleotides and 3'-position modified non-bridged nucleotides.

A number of the nucleotides contained in the central region is 5 to 30, preferably 5 to 15, more preferably 8 to 12, and particularly preferably 9 or 10. A number of the nucleotides contained in the central region is usually selected according to other factors such as strength of the antisense effect to the above-mentioned target RNA, lowness of toxicity, cost, and synthetic yield.

The central region contains at least one sugar moiety-modified nucleotide selected from the group consisting of 2'-3' bridged nucleotides and 3'-position modified non-bridged nucleotides. Next, the 2'-3' bridged nucleotide and the 3'-position modified non-bridged nucleotide contained in the central region will be explained.

The partial structure of the 2'-3' bridged nucleotide contained in the central region is preferably represented by the following formula (I).

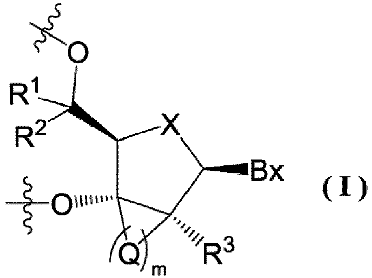

In the formula (I), Bx is a nucleic acid base moiety.

As the nucleic acid base moiety, the above-mentioned "nucleic acid base" can be used.

In the formula (I), X is O or S. X is preferably O.

m is 1, 2, 3 or 4.

-Q-'s are each independently -CR⁴R⁵-, -C(=O)-, -C(=S)-, -C(=NR⁶)-, -O-, -NH-, -NR⁶- or -S-, and when m is 2, 3 or 4, two adjacent -Q-'s may together form a group represented by
the

formula: -CR⁷=CR⁸-.

In the formula (I), R¹, R², R³, R⁴ and R⁵ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano, J¹, J² and J³ are each independently a hydrogen atom or C1-C6 alkyl, Y is O, S or NJ⁴, and J⁴ is C1-C12 alkyl or an amino protective group; R⁶ is C1-C12 alkyl or an amino protective group, and R⁷ and R⁸ are each independently a hydrogen atom or C1-C6 alkyl.

In the formula (I), the nucleic acid base moiety is preferably at least one kind selected from the group consisting of adenine (A), guanine (G), thymine (T), cytosine (C), uracil (U) and 5-methylcytosine (5-me-C). R¹ is preferably a hydrogen atom or C1-C3 alkyl, and more preferably a hydrogen atom. R², R³, R⁴, R⁵, R⁷ and R⁸ are preferably each independently a hydrogen atom or C1-C3 alkyl, and more preferably a hydrogen atom.

R⁶ is preferably C1-C3 alkyl, and more preferably methyl.

In the formula (I), m is preferably 1, 2 or 3, further preferably 2 or 3, and particularly preferably 2.

When m is 2, a partial structure of the preferable 2'-3' bridged nucleotide contained in the central region is represented by the following formula (III).

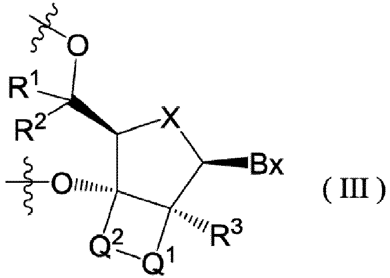

In the formula (III), Bx is a nucleic acid base moiety.

For the nucleic acid base moiety, the above-mentioned "nucleic acid base" can be used.

X is O or S.

-Q¹- and -Q²- are each independently -CR⁴R⁵-, -C(=O)-, -C(=S)-, -C(=NR⁶)-, -O-, -NH-, -NR⁶- or -S-, or, -Q¹-Q²- is -CR⁷=CR⁸-; and wherein R⁷ and R⁸ are each independently a hydrogen atom or C1-C6 alkyl.

R¹, R², R³, R⁴ and R⁵ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano, J¹, J² and J³ are each independently a hydrogen atom or C1-C6 alkyl, Y is O, S or NJ⁴, J⁴ is C1-C12 alkyl or an amino protective group; and R⁶ is C1-C12 alkyl or an amino protective group.

X, Bx and R¹ to R⁸ in the formula (III) have the same meanings as those of X, Bx and R¹ to R⁸ in the formula (I), and preferred embodiments are also the same.

It is preferable that -Q¹- is -O-, -NH-, -NR⁶- or -S-, the R⁶ is C1-C12 alkyl, and -Q²- is -CH₂-, and further preferable that -Q¹- is -O-, and -Q²- is -CH₂-.

The partial structure of the 3'-position modified non-bridged nucleotide contained in the central region is preferably represented by the following formula (II).

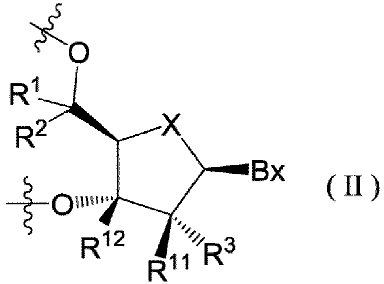

In the formula (II), Bx is a nucleic acid base moiety.

X is O or S.

R¹² is C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano.

R¹, R², R³ and R¹¹ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano; J¹, J² and J³ are each independently a hydrogen atom or C1-C6 alkyl, and Y is O, S or NJ⁴, and J⁴ is C1-C12 alkyl or an amino protective group.

X, Bx and R¹ to R³ in the formula (II) have the same meanings as those of X, Bx and R¹ to R³ in the formula (I), and preferred embodiments are also the same.

R¹¹ is preferably a hydrogen atom or C1-C3 alkyl, and more preferably a hydrogen atom.

R¹² is preferably C1-C6 alkyl or C1-C6 alkyl substituted by one or more substituents, more preferably C1-C3 alkyl, and particularly preferably methyl.

The central region may contain both of the 2'-3' bridged nucleotide and 3'-position modified non-bridged nucleotide. A number (total number) of the 2'-3' bridged nucleotide and 3'-position modified non-bridged nucleotide contained in the central region is 1 to 30, preferably 1 to 5, more preferably 1 to 2, and particularly preferably 1. Numbers of the 2'-3' bridged nucleotide and 3'-position modified non-bridged nucleotide contained in the central region are usually selected according to other factors such as strength of the antisense effect to the above-mentioned target RNA, lowness of toxicity, cost, and synthetic yield.

The 2'-3' bridged nucleotide and 3'-position modified non-bridged nucleotide contained in the central region can be contained in an optional portion of the central region, and preferably contained between the third nucleotide counted from the 3'-end of the central region and the 5'-end thereof. The position at which the 2'-3' bridged nucleotide or 3'-position modified non-bridged nucleotide is contained is usually selected according to other factors such as strength of the antisense effect to the above-mentioned target RNA and lowness of toxicity.

When a portion having SNP is to be a target, in a certain embodiment, it is preferred to contain the 2'-3' bridged nucleotide or the 3'-position modified non-bridged nucleotide in the sequence portion close to a base forming a base pair with a mutated base (for example, within fifth portion, within four portion, within three portion, within two portion or within one portion counted from the base forming a base pair with the mutated base). It is particularly preferable that the base forming a base pair with the mutated base is the 2'-3' bridged nucleotide or 3'-position modified non-bridged nucleotide.

Among the nucleotides contained in the central region, it is preferable that at least one of the nucleotides is phosphorothioated, further preferably 80% of the nucleotides is phosphorothioated, further more preferably 90% of the nucleotides is phosphorothioated, and particularly preferably all are phosphorothioated.

The 5'-side region comprises at least one nucleotide independently selected from the group consisting of deoxyribonucleotides, ribonucleotides and sugar moiety-modified nucleotides, and the 3'-terminal thereof is a sugar moiety-modified nucleotide, where the sugar moiety-modified nucleotide at the 3'-terminal binds to the central region, and selected from the sugar moiety-modified nucleotides excluding a 2'-3' bridged nucleotide and 3'-position-modified non-bridged nucleotide, and does not contain an oligonucleotide strand constituted by at least four contiguous nucleotides which are independently selected from the group consisting of deoxyribonucleotides, 2'-3' bridged nucleotides and 3'-position-modified non-bridged nucleotides.

The 3'-side region comprises at least one nucleotide independently selected from the group consisting of deoxyribonucleotides, ribonucleotides and sugar moiety-modified nucleotides, and the 5'-terminal thereof is a sugar moiety-modified nucleotide, where the sugar moiety-modified nucleotide at the 5'-terminal binds to the central region, and selected from the sugar moiety-modified nucleotides excluding a 2'-3' bridged nucleotide and 3'-position modified non-bridged nucleotide, and does not contain an oligonucleotide strand constituted by at least four contiguous nucleotides which are independently selected from the group consisting of deoxyribonucleotides, 2'-3' bridged nucleotides and 3'-position modified non-bridged nucleotides.

The number of the nucleotides contained in the 5'-side region is 1 to 15, preferably 1 to 7, more preferably 2 to 5, and particularly preferably 3. The number of the nucleotides contained in the 5'-side region is usually selected according to other factors such as strength of the antisense effect to the above-mentioned target RNA, lowness of toxicity, cost, and synthetic yield. The 3'-side region is the same as in the 5'-side region.

The sugar moiety-modified nucleotide contained in the 5'-side region is preferably a nucleotide in which affinity for a partial sequence of the target RNA is increased or a nucleotide in which resistance to a nuclease is increased, by substitution or the like. It is more preferably independently selected from a 2'-position modified non-bridged nucleotide and 2',4'-BNA.

The 2'-position modified non-bridged nucleotide is preferably independently selected from the group consisting of 2'-O-methyl nucleotides, 2'-O-methoxyethyl (MOE) nucleotides, 2'-O-aminopropyl (AP) nucleotides, 2'-fluoronucleotides, 2'-O-(N-methylacetamido) (NMA) nucleotides and 2'-O-methylcarbamoylethyl (MCE) nucleotides, more preferably independently selected from 2'-O-methoxyethyl (MOE) nucleotides and 2'-O-methylcarbamoylethyl (MCE) nucleotides, and is particularly preferably 2'-O-methoxyethyl (MOE) nucleotides.

The 2',4'-BNA is preferably LNA, cEt-BNA, ENA, BNA^{NC}, AmNA and scpBNA, more preferably LNA containing a partial structure represented by the following formula (VI). The 3'-side region is the same as in the 5'-side region.

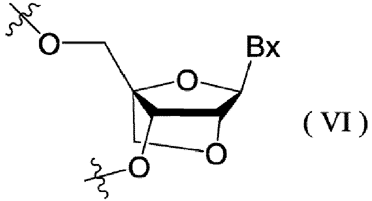

In the formula, Bx represents a nucleic acid base moiety, and has the same meaning as Bx in the formula (I).

The types, numbers and locations of the sugar moiety-modified nucleotide, deoxyribonucleotide and ribonucleotide in the 5'-side region can have an effect on the antisense effect and the like demonstrated by the antisense oligonucleotide disclosed herein. Although the types, numbers and locations thereof are unable to be unconditionally defined since they differ according to the sequence and so forth of the target RNA, and thus cannot be generally stated, the persons of ordinary skill in the art are able to determine a preferable aspect thereof while referring to the above-mentioned descriptions in the literature relating to antisense methods. In addition, if the antisense effect demonstrated by the oligonucleotide after modification of a base moiety, sugar moiety or phosphodiester bond moiety is measured and the resulting measured value is not significantly lowered than that of the oligonucleotide prior to modification (such as if the measured value of the oligonucleotide after modification is 30% or more of the measured value of the oligonucleotide prior to modification), then that modification can be evaluated as a preferable aspect. Measurement of antisense effect can be carried out, as is indicated in, for example, the examples to be subsequently described, by introducing a test oligonucleotide into a cell and the like, and measuring the expression level of the target RNA, expression level of cDNA associated with the target RNA or the amount of a protein associated with the target RNA, which is controlled by the antisense effect demonstrated by the test oligonucleotide optionally using a known technique such as northern blotting, quantitative PCR or western blotting. The 3'-side region is the same as in the 5'-side region.

A preferred embodiment in the 5'-side region is an oligonucleotide comprising 2 to 5 nucleotides independently selected from the group consisting of 2'-position modified non-bridged nucleotides, 2',4'-BNA, and deoxyribonucleotides, and contains at least two nucleotides selected from the group consisting of 2'-position modified non-bridged nucleotide and 2',4'-BNA. More preferably, it is an oligonucleotide comprising 2 to 5 nucleotides independently selected from the group consisting of 2'-position modified non-bridged nucleotides and 2',4'-BNA, and further preferably, it is an oligonucleotide comprising 2 to 3 nucleotides independently selected from the group consisting of 2'-position modified non-bridged nucleotides and 2',4'-BNA. Further preferably, it is an oligonucleotide comprising 2 to 3 nucleotides independently selected from the group consisting of LNA and 2'-O-methoxyethyl (MOE) nucleotides, and particularly preferably, it is an oligonucleotide comprising 2 to 3 LNAs.

As another preferred embodiment, it is an oligonucleotide comprising five 2'-position modified non-bridged nucleotides.

As still other preferred embodiment, the 5'-side region comprises 2 to 5 nucleotides independently selected from the group consisting of 2',4'-BNA and deoxyribonucleotides, and is an oligonucleotide containing at least two 2',4'-BNAs, and such an oligonucleotide can be referred to WO 2016/127002. The 3'-side region is the same as in the 5'-side region.

Among the nucleotides contained in the 5'-side region, at least one nucleotide is preferably phosphorothioated, further preferably 50% of the nucleotides are phosphorothioated, further more 80% of the nucleotide are phosphorothioated, and particularly preferably all are phosphorothioated. As another preferred embodiment, all of the nucleotides contained in the 5'-side region are preferably linked by a phosphodiester bond. The 3'-side region is the same as in the 5'-side region.

In the antisense oligonucleotide of the present invention, the 3'-end of the 5'-side region and the 5'-end of the central region are linked by forming a phosphodiester bond or a modified phosphodiester bond, the 5'-end of the 3'-side region and the 3'-end of the central region are linked by forming a phosphodiester bond or a modified phosphodiester bond. Preferably, the 3'-end of the 5'-side region and the 5'-end of the central region are linked by forming a modified phosphodiester bond, and the 5'-end of the 3'-side region and the 3'-end of the central region are linked by forming a modified phosphodiester bond. Further preferably, the 3'-end of the 5'-side region and the 5'-end of the central region are linked by a phosphorothioate bond, and the 5'-end of the 3'-side region and the 3'-end of the central region are linked by forming a phosphorothioate bond.

A functional molecule may be bound directly or indirectly to the antisense oligonucleotide of the present invention. The bonding between the functional molecule and the antisense oligonucleotide may be directly or indirectly through the other substance, and the oligonucleotide and the functional molecule are preferably bound through a covalent bond, an ionic bond or a hydrogen bond. From the viewpoint of high bond stability, they are more preferably bound directly through a covalent bond or bound with a linker (a linking group) through a covalent bond.

In the case the above-mentioned functional molecule is bound to the antisense oligonucleotide by a covalent bond, the above-mentioned functional molecule is preferably bound directly or indirectly to the 3'-end or 5'-end of the antisense oligonucleotide molecule. Bonding between the above-mentioned linker or the functional molecule and the terminal nucleotide of the antisense oligonucleotide molecule is selected according to the functional molecule.

The above-mentioned linker or the functional molecule and the terminal nucleotide of the antisense oligonucleotide molecule are preferably coupled through a phosphodiester bond or a modified phosphodiester bond, and more preferably coupled through a phosphodiester bond.

The above-mentioned linker or functional molecule may be directly coupled with an oxygen atom at the 3'-position possessed by the nucleotide at the 3'-end or an oxygen atom at the 5' - position possessed by the nucleotide at the 5'-end of the antisense oligonucleotide molecule.

The structure of the "functional molecule" is not particularly limited, and a desired function is imparted to the antisense oligonucleotide as a result of bonding therewith. As the desired functions, there may be mentioned a labeling function, purifying function and delivering function to a target site. Examples of molecules that impart a labeling function may be mentioned fluorescent proteins and compounds such as luciferase. Examples of molecules that impart a purifying function may be mentioned compounds such as biotin, avidin, His-tag peptide, GST-tag peptide or FLAG-tag peptide.

In addition, from the viewpoint of efficiently delivering an antisense oligonucleotide to a target site (for example, a target cell) with high specificity and efficiently, and extremely effectively suppressing expression of a target gene with the antisense oligonucleotide, a molecule having a function that causes the antisense oligonucleotide to be delivered to a target site is preferably bound as a functional molecule. The molecules having such a delivery function can be referred to, for example, European Journal of Pharmaceutics and Biopharmaceutics, 2016, vol. 107, pp. 321-340, Advanced Drug Delivery Reviews, 2016, vol. 104, pp. 78-92, and Expert Opinion on Drug Delivery, 2014, vol. 11, pp. 791-822.

As the molecule that impart a delivery function to target RNA, there may be mentioned lipids and sugars from the viewpoint of, for example, being able to efficiently deliver an antisense oligonucleotide to the liver and the like with high specificity and efficiently. Such lipids may be mentioned cholesterol; fatty acids; fat-soluble vitamins such as vitamin E (tocopherols, tocotrienols), vitamin A, vitamin D and vitamin K; intermediate metabolites such as acylcarnitine and acyl CoA; glycolipids; glycerides; and derivatives thereof. Among these, cholesterol and vitamin E (tocopherols, tocotrienols) are preferable from the viewpoint of higher safety. Above all, tocopherols are more preferable, tocopherol is further preferable, and α-tocopherol is particularly preferable. As the sugars, sugar derivatives that interact with asialoglycoprotein receptor are mentioned.

"Asialoglycoprotein receptors" are present on the surface of liver cells and have an action that recognizes a galactose residue of an asialoglycoprotein and incorporates the molecules into the cell where they are degraded. "Sugar derivatives that interact with asialoglycoprotein receptors" are preferably compounds that have a structure similar to the galactose residue and are incorporated into cells due to interaction with asialoglycoprotein receptors, and may be mentioned, for example, GalNAc (N-acetylgalactosamine) derivatives, galactose derivatives and lactose derivatives. In addition, from the viewpoint of being able to efficiently deliver the antisense oligonucleotide of the present invention to the brain with high specificity, as the "functional molecules", there may be mentioned sugars (for example, glucose and sucrose). In addition, from the viewpoint of being able to efficiently deliver the antisense oligonucleotide to the various organs with high specificity by interacting with various proteins on the cell surface of the respective organs, as the "functional molecules", there may be mentioned receptor ligands, antibodies, and peptides or proteins of fragments thereof.

Since the linker used to intermediate bonding between a functional molecule and an antisense oligonucleotide is only required to be able to demonstrate the function possessed by the functional molecule as an antisense oligonucleotide molecule, it is not particularly limited as long as it is a linker that can stably bond the functional molecule and the oligonucleotide. As the linker, there may be mentioned, for example, a group derived from oligonucleotides having a number of the nucleotides of 1 to 20, a group derived from polypeptides having a number of the amino acids of 2 to 20, alkylene having 2 to 20 carbon atoms and alkenylene having 2 to 20 carbon atoms. The above-mentioned group derived from oligonucleotides having a number of the nucleotide of 2 to 20 is a group in which hydroxy or a hydrogen atom is removed from the oligonucleotide having a number of the nucleotides of 2 to 20. The above-mentioned group derived from oligonucleotides having a number of the nucleotides of 1 to 20 can be referred to, for example, WO 2017/053995. In WO 2017/053995, there is described, for example, a linker with 3 bases having a TCA motif, and a linker with 1 to 5 bases having no TCA motif. The above-mentioned group derived from polypeptides having a number of the amino acids of 2 to 20 is a group in which hydroxy, a hydrogen atom or amino is removed from the polypeptide having a number of the amino acids of 2 to 20.

The linker is preferably C2-C20 alkylene or C2-C20 alkenylene (methylenes contained in the alkylene and alkenylene are each independently unsubstituted, or substituted by 1 or 2 substituents selected from the group consisting of a halogen atom, hydroxy, protected hydroxy, oxo and thioxo. In addition, methylenes of the alkylene and alkenylene are each independently not replaced, or replaced with -O-, -NR^{B}- (R^{B} is a hydrogen atom, C1-C6 alkyl or halo-C1-C6 alkyl), -S-, -S(=O)- or -S(=O)₂-). Here, by combining the above-mentioned substitutions and replacements, the linker may also contain a group represented by -C(=O)-O-, -O-C(=O)-NR¹³- (R¹³ represents a hydrogen atom, C1-C6 alkyl or halo-C1-C6 alkyl), -C(=O)-NR¹³- (R¹³ represents a hydrogen atom, C1-C6 alkyl or halo-C1-C6 alkyl), -C(=S)-NR¹³- (R¹³ represents a hydrogen atom, C1-C6 alkyl or halo-C1-C6 alkyl), or -NR¹³-C(=O)-NR¹³- (R¹³s each independently represents a hydrogen atom, C1-C6 alkyl or halo-C1-C6 alkyl).

The linker is more preferably C2-C20 alkylene (methylenes of the alkylene are each independently not replaced, or replaced with -O-. The methylenes not replaced are each independently unsubstituted, or substituted by hydroxy or protected hydroxy), further preferably C8-C12 alkylene (methylenes of the alkylene are each independently not replaced, or replaced with -O-. The methylenes not replaced are each independently unsubstituted, or substituted by hydroxy), and particularly preferably 1,8-octylene. In addition, as another aspect thereof, the linker is particularly preferably a group represented by the following formula (VII).

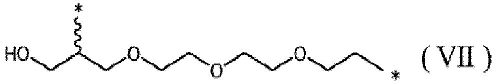

In the formula, one asterisk * represents a bonding site (an atom that composes a nucleotide) with a group derived from those oligonucleotides, while the other asterisk * represents a bonding site (an atom that constitutes a group derived from a functional molecule) with a group derived from a functional molecule.

As another aspect thereof, the linker is more preferably C2-C20 alkylene (methylenes of the alkylene are each independently not replaced, or replaced with -O- or -NR^{B}- (R^{B} is a hydrogen atom or C1-C6 alkyl). The methylenes not replaced are each independently unsubstituted or substituted by oxo), further preferably a group represented by the following formula:

-N(H)C(=O)-(CH₂)ₑ-N(H)C(=O)-(CH₂)ₑ-C(=O)-

(wherein e's are each independently an integer of 1 to 6), and particularly preferably a group represented by the following formula:

-N(H)C(=O)-(CH₂)ₑ-N(H)C(=O)-(CH₂)ₑ-C(=O)-

A protective group of the above-mentioned "protected hydroxy" is not particularly limited since it may be stable at the time of bonding the functional molecule and the oligonucleotide. The linker is not particularly limited and may be mentioned an optional protective group described in, for example, Protective Groups in Organic Synthesis 4th Edition, written by T. W. Greene, and P. G. M. Wuts, John Wiley & Sons Inc.(2006). Specifically, there may be mentioned C1-C6 alkyl (for example, there may be mentioned methyl and t-butyl), ether-based protective groups such as triarylmethyl (for example, there may be mentioned triphenylmethyl (trityl), monomethoxytrityl, dimethoxytrityl (DMTr) and trimethoxytrityl); acetal-based protective groups such as methoxymethyl, methylthiomethyl, methoxyethyl, benzyloxymethyl, 2-tetrahydropyranyl and ethoxyethyl; acyl-based protective groups such as acyl (for example, there may be mentioned formyl, acetyl, pivaloyl and benzoyl); silyl-based protective groups such as tri(C1-C6 alkyl)silyl (for example, there may be mentioned trimethylsilyl, triethylsilyl, triisopropylsilyl, t-butyldimethylsilyl and dimethylisopropylsilyl), (C1-C6 alkyl)diarylsilyl (for example, there may be mentioned t-butyldiphenylsilyl and diphenylmethylsilyl), triarylsilyl (for example, there may be mentioned triphenylsilyl), tribenzylsilyl and [(triisopropylsilyl)oxy]methyl (Tom group); 1-(4-chlorophenyl)-4-ethoxypiperidin-4-yl (Cpep group), 9-phenylxanthen-9-yl (Pixyl group) and 9-(p-methoxyphenyl)xanthen-9-yl (MOX group). A protective group of the "protected hydroxy" is preferably benzoyl, trimethylsilyl, triethylsilyl, triisopropylsilyl, t-butyldimethylsilyl, triphenylmethyl, monomethoxytrityl, dimethoxytrityl, trimethoxytrityl, 9-phenylxanthen-9-yl or 9-(p-methoxyphenyl)xanthen-9-yl, more preferably, monomethoxytrityl, dimethoxytrityl or trimethoxytrityl, and further more preferably dimethoxytrityl.

In the present invention, a prodrug of the antisense oligonucleotide is also contained.

A prodrug refers to a derivative of a pharmaceutical compound having a group that can be chemically or metabolically degraded, and is a compound that is degraded by solvolysis or in vivo under physiological conditions and derived to a pharmacologically active pharmaceutical compound. Suitable method for selecting and method for producing prodrug derivatives are described in, for example, Design of Prodrugs(Elsevier, Amsterdam, 1985). In the case of the present invention, and in the case of having a hydroxy group, there may be exemplified by a prodrug such as acyloxy derivatives produced by reacting the compound with a suitable acyl halide, a suitable acid anhydride or a suitable halogenated alkyloxycarbonyl compound. As the prodrug and the particularly preferable structure, there may be mentioned -O-C(=O)C₂H₅, -O-C(=O)(t-Bu), -O-C(=O)C₁₅H₃₁, -O-C(=O)-(m-CO₂Na-Ph), -O-C(=O)CH₂CH₂CO₂Na-OC(=O)CH(NH₂)CH₃, -O-C(=O)CH₂N(CH₃)₂ or -O-CH₂OC(=O)CH₃. In the case the antisense oligonucleotide that forms the present invention has an amino group, there may be exemplified by a prodrug produced by reacting the compound having an amino group with a suitable acid halide, a suitable mixed acid anhydride or a suitable halogenated alkyloxycarbonyl compound. As the prodrug and the particularly preferable structure, there may be mentioned -NH-C(=O) - (CH₂)₂₀OCH₃, -NH-C(=O)CH(NH₂)CH₃ and -NH-CH₂OC(=O)CH₃.

Another preferable structure of the prodrug included in the present invention may be mentioned double-stranded oligonucleotides (for example, there are described in WO 2013/089283, WO 2017/068791, WO 2017/068790 or WO 2018/003739) containing oligonucleotides (for example, oligoribonucleotide nucleotide, RNA) which contain ribonucleotide, oligonucleotides which contain peptide nucleic acids (PNA), or oligonucleotides (for example, oligodeoxyribonucleotide, DNA) which contain deoxyribonucleotides, which are complementary to an antisense oligonucleotide, and single-stranded oligonucleotides (for example, there is described in WO 2017/131124) in which RNA oligonucleotides complementary to an antisense oligonucleotide are linked by a linker. The linker is not limited only to those described in WO 2017/131124 and may contain, for example, a non-nucleotide structure. In addition, there may be mentioned single-stranded oligonucleotides in which RNA oligonucleotides complementary to an antisense oligonucleotide are directly linked.

More specific examples of the prodrug of the present invention may be mentioned below.

### (A)

An oligonucleotide complex comprising
(i) the above-mentioned antisense oligonucleotide, and
(ii) an oligonucleotide containing at least one ribonucleotide, and containing a region which hybridizes with the above-mentioned (i) antisense oligonucleotide.

### (B)

An oligonucleotide which contains
(i) a group derived from the above-mentioned antisense oligonucleotide, and
(ii) a group derived from an oligonucleotide which contains at least one ribonucleotide, and contains a region that hybridizes with the above-mentioned (i) antisense oligonucleotide, and
the group derived from the above-mentioned (i) the antisense oligonucleotide, and the above-mentioned (ii) group derived from the oligonucleotide
are linked.

In (B), (i) the group derived from the antisense oligonucleotide, and (ii) the group derived from an oligonucleotide may be linked by a group derived from an oligonucleotide which is degraded under physiological conditions, may be linked by a linking group containing a non-nucleotide structure, or may be linked directly.

### (C)

An oligonucleotide complex which contains
(iii) an oligonucleotide in which an oligonucleotide strand containing at least one ribonucleotide is linked to the above-mentioned group derived from the antisense oligonucleotide, and
(iv) an oligonucleotide containing an oligonucleotide strand which contains at least four contiguous nucleotides recognized by RNase H,
wherein the above-mentioned oligonucleotide strand containing at least one ribonucleotide of the above-mentioned (iii), and the above-mentioned oligonucleotide strand containing at least four contiguous nucleotides recognized by RNase H of the above-mentioned (iv) are hybridized.

### (D)

An oligonucleotide which contains
(iii) a group derived from an oligonucleotide containing an oligonucleotide in which an oligonucleotide strand containing at least one ribonucleotide is linked with a group derived from the above-mentioned antisense oligonucleotide, and
(iv) a group derived from an oligonucleotide which contains an oligonucleotide strand containing at least four contiguous nucleotides recognized by RNase H,
wherein the group derived from the oligonucleotide of the above-mentioned (iii), and the group derived from the oligonucleotide of the above-mentioned (iv) are linked, and the oligonucleotide strand containing at least one ribonucleotide of the above-mentioned (iii) and the oligonucleotide strand containing at least four contiguous nucleotides recognized by RNase H of the above-mentioned (iv) are hybridized.

In (D), (iii) the group derived from the oligonucleotide, and (iv) the group derived from the oligonucleotide may be linked by a group derived from oligonucleotide which is degraded under physiological conditions, may be linked by a linking group containing a non-nucleotide structure, or may be linked directly.

In (C) and (D), the group derived from the antisense oligonucleotide and the oligonucleotide strand containing at least one ribonucleotide may be linked by a group derived from oligonucleotide which is degraded under physiological conditions, may be linked by a linking group containing a non-nucleotide structure, or may be linked directly.

The "oligonucleotide degradable under physiological conditions" may be any oligonucleotide degradable by enzymes such as various kinds of DNase (deoxyribonuclease) and RNase (ribonuclease) under physiological conditions, and a base, sugar or phosphate bond of the nucleotides constituting the oligonucleotide may be or may not be chemically modified in all or a portion thereof. The "oligonucleotide degradable under physiological conditions" may be, for example, an oligonucleotide containing at least one phosphodiester bond, preferably linked by the phosphodiester bond, more preferably an oligodeoxyribonucleotide or an oligoribonucleotide, further preferably DNA or RNA, and further more preferably RNA.

The oligonucleotide degradable under physiological conditions may contain or may not contain a partially complementary sequence in the oligonucleotide degradable under physiological conditions, preferably does not contain partially complementary sequence. Examples of such an oligonucleotide may be mentioned (N)_{k'}(N's each independently represent adenosine, uridine, cytidine, guanosine, 2'-deoxyadenosine, thymidine, 2'-deoxycytidine, or 2'-deoxyguanosine, and k is an integer (repeating number) of 1 to 40) linked by a phosphodiester bond. Among these, k' is preferably 3 to 20, more preferably 4 to 10, further preferably 4 to 7, further more preferably 4 or 5, and particularly preferably 4.

A "linking group containing a non-nucleotide structure" is a linking group having at least one "non-nucleotide structure" as a constitutional unit. As the non-nucleotide structure, there may be mentioned, for example, a structure having no nucleic acid base. The "linking group containing a non-nucleotide structure" may contain a nucleotide (a deoxyribonucleoside group, a ribonucleoside group, etc.), and may not contain the same. The "linking group containing a non-nucleotide structure" is, for example, a group of the following structure.

-[P¹¹-(-O-V¹¹-)q₁₁-O-]q₁₂-P¹¹-

{wherein V¹¹ is
C2-C50 alkylene
(the C2-C50 alkylene is unsubstituted or substituted by one or more substituents independently selected from the substituent group V^{a}),
a group selected from the group consisting of the following formulae (XIII-1) to (XIII-11): 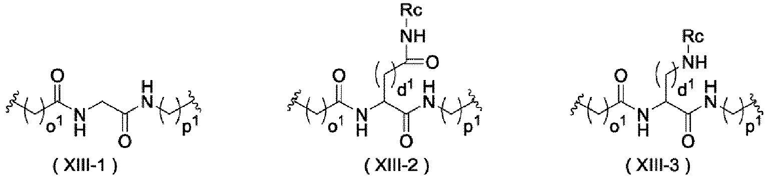 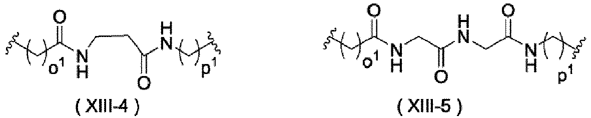 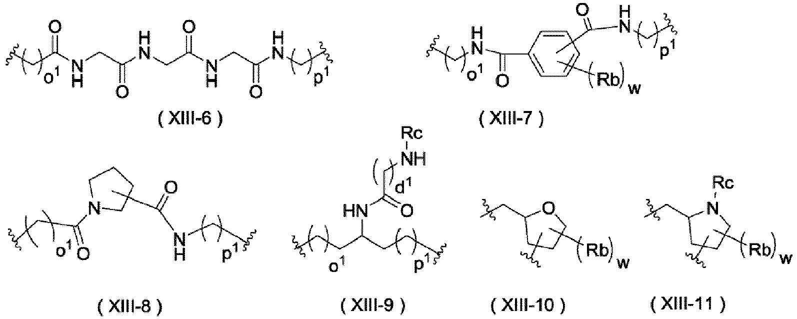
(wherein o¹ is an integer of from 0 to 30, p¹ is an integer of from 0 to 30, d¹ is an integer of from 1 to 10, w is an integer of from 0 to 3, Rb is a halogen atom, hydroxy, amino, C1-C6 alkoxy, C1-C6 alkoxy substituted by C1-C6 alkoxy or carbamoyl, mono-C alkylamino, di-C1-C6 alkylamino or C alkyl group, Rc is a hydrogen atom, a C1-C6 alkyl, halo-C1-C6 alkyl, C1-C6 alkylcarbonyl, halo-C1-C6 alkylcarbonyl, C1-C6 alkoxycarbonyl, C1-C6 alkoxycarbonyl substituted by C1-C6 alkoxy or carbamoyl, mono-C 1-C6 alkylaminocarbonyl, di-C1-C6 alkylaminocarbonyl, C1-C6 alkylsulfonyl, halo-C1-C6 alkylsulfonyl, C1-C6 alkoxysulfonyl, C1-C6 alkoxysulfonyl substituted by C1-C6 alkoxy or carbamoyl, mono-C 1-C6 alkylaminosulfonyl or di-C1-C6 alkylaminosulfonyl),
   the ribonucleoside group, or
   the deoxyribonucleoside group,
at least one of V¹¹s is a group selected from C2-C50 alkylene (the C2-C50 alkylene is unsubstituted or substituted by one or more substituents independently selected from the substituent group V^{a}), or the above-mentioned formulae (XIII-1) to (XIII-11),
the substituent group V^{a} refers to a substituent group constituted by hydroxy, a halogen atom, cyano, nitro, amino, carboxy, carbamoyl, sulfamoyl, phosphono, sulfo, tetrazolyl and formyl,
P¹¹ are each independently -P(=O)(OH)- or -P(=O)(SH)-,
at least one of P¹¹s is -P(=O)(OH)-,
q₁₁ is an integer of from 1 to 10, q₁₂ is an integer of from 1 to 20, and when at least one of q₁₁ and q₁₂ is 2 or more, V¹¹s are the same or different from each other.}

Here, o¹ is preferably an integer of from 1 to 30, p¹ is preferably an integer of from 1 to 30. q₁₁ is preferably an integer of from 1 to 6, and more preferably an integer of from 1 to 3. q₁₂ is preferably an integer of from 1 to 6, and more preferably an integer of from 1 to 3. P¹¹ is preferably -P(=O)(OH)-.

Hereinafter, explanations will be made with regard to the oligonucleotide of (ii) in the above-mentioned (A), groups derived from those oligonucleotides of (ii) in the above-mentioned (B), and the oligonucleotide strand containing at least one ribonucleotide (the portion which hybridizes with the oligonucleotide strand containing at least four contiguous nucleotides recognized by RNase H) among the oligonucleotides of (iii) in the oligonucleotide or oligonucleotide complex shown in the above-mentioned (C) and (D).

The types, numbers and locations of sugar moiety-modified nucleotides, deoxyribonucleotides and ribonucleotides can have an effect on the antisense effect and the like demonstrated by the prodrug of the antisense oligonucleotide disclosed herein. Although the types, numbers and locations thereof are unable to be unconditionally defined since they differ according to the sequence and so forth of the target RNA, the persons of ordinary skill in the art are able to determine a preferable aspect thereof while referring to the above-mentioned descriptions in the literature relating to antisense methods. In addition, if the antisense effect demonstrated by the prodrug of the antisense oligonucleotide after modification of a base moiety, sugar moiety or phosphodiester bond moiety is measured and the resulting measured value is not significantly lower than that of the prodrug of the antisense oligonucleotide prior to modification (such as if the measured value of the prodrug of the antisense oligonucleotide after modification is 30% or more of the measured value of the prodrug prior to modification), then that modification can be evaluated as a preferable aspect. As is indicated in, for example, Examples to be subsequently described, measurement of the antisense effect can be carried out by introducing a test oligonucleotide into a cell and the like, and measuring the expression level of target RNA, expression level of cDNA associated with the target RNA or the amount of a protein associated with the target RNA, which is controlled by the antisense effect demonstrated by the test oligonucleotide, optionally using a known technique such as northern blotting, quantitative PCR or western blotting.

The oligonucleotide of (ii) in the oligonucleotide complex shown in the above-mentioned (A) or the group derived from the oligonucleotide of (ii) in the oligonucleotide shown in the above-mentioned (B) is independently selected from ribonucleotides, deoxyribonucleotides and sugar moiety-modified nucleotides, and preferably selected from ribonucleotides. When the oligonucleotide or group derived from the oligonucleotide of (ii) is selected from ribonucleotides, the ribonucleotide is preferably linked to each other by the phosphodiester bond.

As another embodiment, the oligonucleotide or group derived from the oligonucleotide of (ii) is selected from ribonucleotides and sugar moiety-modified nucleotides, and the sugar moiety-modified nucleotide is selected from sugar moiety-modified nucleotides excluding 2'-3' bridged nucleotides and 3'-position modified non-bridged nucleotides. At this time, it is preferable that the end of the oligonucleotide is at least one sugar moiety-modified nucleotide. This sugar moiety-modified nucleotide is preferably a 2'-O-methylated nucleotide, and is preferably bonded to an adjacent nucleotide by a phosphorothioate bond. In the oligonucleotide complex shown in the above-mentioned (C) or the oligonucleotide shown in (D), the oligonucleotide strand containing at least one ribonucleotide (the portion which hybridizes with the oligonucleotide strand containing at least four contiguous nucleotides recognized by RNase H) among the oligonucleotides of (iii) is the same.

In the case of (A), the nucleotides at the 3'-end and the 5'-end of the oligonucleotide of (ii) are preferably sugar moiety-modified nucleotides. In the case of (B), among the 3'-end and the 5'-end of the groups derived from the oligonucleotides of (ii), the terminal nucleotide not bound to (i) the group derived from the antisense oligonucleotide is preferably a sugar moiety-modified nucleotide. In the case of (C) and (D), among the 3'-end and the 5'-end of the groups derived from the oligonucleotides of (iii), the terminal nucleotide not bound to the above-mentioned group derived from the antisense oligonucleotide is preferably a sugar moiety-modified nucleotide.

The number of the bases of the oligonucleotide or group derived from the oligonucleotides of (ii) is not particularly limited, and may be the same as or different from the number of the bases of the (i) antisense oligonucleotide (or a group derived from). In (A), the numbers of the bases of the oligonucleotides of (i) and (ii) are preferably the same, and all of the oligonucleotides of (i) and (ii) are preferably hybridized. The same applies in (B), when the groups derived from the oligonucleotides of (i) and (ii) are linked by a group derived from oligonucleotide which is degraded under physiological conditions or a linking group containing a non-nucleotide structure.

The oligonucleotides of (iv) in the oligonucleotide complex shown in the above-mentioned (C), and the groups derived from the oligonucleotides of (iv) in the oligonucleotide in the above-mentioned (D) are independently selected from ribonucleotides, deoxyribonucleotides and sugar moiety-modified nucleotides, and are preferably selected from deoxyribonucleotides and sugar moiety-modified nucleotides. The sugar moiety-modified nucleotides contained in the oligonucleotides or the groups derived from the oligonucleotides of (iv) are preferably selected from the sugar moiety-modified nucleotides excluding the 2'-3' bridged nucleotides and 3'-position modified non-bridged nucleotides. At this time, the 5'-end and 3'-end of the oligonucleotides or the groups derived from the oligonucleotides are preferably at least one sugar moiety-modified nucleotide. The at least one sugar moiety-modified nucleotides are preferably at least one selected from 2'-position modified non-bridged nucleotides and 2',4'-BNA, and more preferably at least one selected from the group consisting of 2'-O-methyl nucleotide, 2'-O-methoxyethyl (MOE) nucleotide, 2'-O-aminopropyl (AP) nucleotide, 2'-fluoronucleotide, 2'-O-(N-methylacetamido) (NMA) nucleotide, 2'-O-methylcarbamoylethyl (MCE) nucleotide, LNA, cEt-BNA, ENA, BNA^{NC}, AmNA and scpBNA. The nucleotides contained in the oligonucleotides of (iv) or the groups derived from the oligonucleotides are preferably linked to each other by a phosphorothioate bond.

In the above-mentioned (A),
it can be considered that the portion containing the above-mentioned (i) antisense oligonucleotide and (ii) at least one ribonucleotide, and the region which hybridizes with the above-mentioned (i) antisense oligonucleotide are hybridized is recognized by RNase H, and the region containing (ii) at least one ribonucleotide, and hybridizes with the above-mentioned (i) antisense oligonucleotide is cleaved. As a result, in the target cell and the like, the antisense oligonucleotide of the present invention is produced, and the prodrug of (A) is considered to exert a therapeutic effect and the like. The same applies to the above-mentioned (B).

In the above-mentioned (C),
it can be considered that a portion in which the above-mentioned oligonucleotide strand containing at least one of ribonucleotides of the above-mentioned (iii), and the above-mentioned oligonucleotide strand containing at least four contiguous nucleotides recognized by RNase H of the above-mentioned (iv) are hybridized, is recognized by RNase H, and the above-mentioned oligonucleotide strand containing at least one of ribonucleotides of (iii) is cleaved. As a result, in the target cell and the like, the antisense oligonucleotide of the present invention is produced, and the prodrug of (C) is considered to exert a therapeutic effect and the like. The same applies to the above-mentioned (D).

When the oligonucleotide complex shown in the above-mentioned (A) has a functional molecule, the oligonucleotide of (ii) preferably contains a functional molecule, and the functional molecule is preferably bound to the end of the oligonucleotide of (ii). The same applies to the oligonucleotide shown in the above-mentioned (B). When the oligonucleotide complex shown in the above-mentioned (C) has a functional molecule, the oligonucleotide of (iv) preferably contains a functional molecule, and the functional molecule is preferably bound to the end of the oligonucleotide of (iv). The same applies to the oligonucleotide shown in the above-mentioned (D). Preferred embodiments of the functional molecule and its binding are as described above.

Among the above-mentioned (A) and (B), the oligonucleotide or the group derived from the oligonucleotides of (ii) may further have a group derived from the antisense oligonucleotide. The group derived from the antisense oligonucleotide of the (ii) may be the same as or different from the antisense oligonucleotide or the group derived from the oligonucleotides of (i). Also, it may be or may not be the group derived from the antisense oligonucleotide of the present invention. The group derived from the antisense oligonucleotide of the above-mentioned (ii) preferably does not hybridize with the antisense oligonucleotide or the group derived from the antisense oligonucleotide of (i).

Among the above-mentioned (C) and (D), the oligonucleotide or the group derived from the oligonucleotides of (iv) may be the antisense oligonucleotide or the group derived from the antisense oligonucleotide. The antisense oligonucleotide or the group derived from the antisense oligonucleotide of the (iv) may be the same as or different from the group derived from the antisense oligonucleotide contained in the oligonucleotide of (iii). Also, it may be or may not be the group derived from the antisense oligonucleotide of the present invention. The antisense oligonucleotide or the group derived from the antisense oligonucleotide of the above-mentioned (iv) preferably does not hybridize with the antisense oligonucleotide or the group derived from the antisense oligonucleotide of (iii).

As an antisense oligonucleotide which is not the antisense oligonucleotide of the present invention, for example, the following antisense oligonucleotides are mentioned.
(1) An antisense oligonucleotide having a central region, a 5'-side region and a 3'-side region, wherein
   - the central region
      comprises at least 5 nucleotides independently selected from the group consisting of deoxyribonucleotides, ribonucleotides and sugar moiety-modified nucleotides, the above-mentioned sugar moiety-modified nucleotide is selected from a sugar moiety-modified nucleotide excluding a 2'-3' bridged nucleotide and 3'-position modified non-bridged nucleotide,
      the 3'-end and the 5'-end are each independently a deoxyribonucleotide or ribonucleotide, and
      contain at least one of an oligonucleotide strand constituted by at least four contiguous nucleotides which are independently selected from deoxyribonucleotides;
   - the 5'-side region
      comprises at least one nucleotide independently selected from the group consisting of deoxyribonucleotides, ribonucleotides and sugar moiety-modified nucleotides, and the 3'-terminal thereof is a sugar moiety-modified nucleotide, where the sugar moiety-modified nucleotide at the 3'-terminal binds to the central region, and selected from sugar moiety-modified nucleotides excluding 2'-3' bridged nucleotides and 3'-position modified non-bridged nucleotides, and
      does not contain an oligonucleotide strand constituted by at least four contiguous nucleotides which are independently selected from the group consisting of deoxyribonucleotides, 2'-3' bridged nucleotides and 3'-position modified non-bridged nucleotides; and
   - the 3'-side region
      comprises at least one nucleotide independently selected from the group consisting of deoxyribonucleotides, ribonucleotides and sugar moiety-modified nucleotides, and the 5'-terminal thereof is a sugar moiety-modified nucleotide, where the sugar moiety-modified nucleotide at the 5'-terminal binds to the central region, and selected from sugar moiety-modified nucleotides excluding 2'-3' bridged nucleotides and 3'-position modified non-bridged nucleotides, and
      does not contain an oligonucleotide strand constituted by at least four contiguous nucleotides which are independently selected from the group consisting of deoxyribonucleotides, 2'-3' bridged nucleotides and 3'-position modified non-bridged nucleotides.

   Among these, there may be mentioned the antisense oligonucleotide of the following (2).
(2) An antisense oligonucleotide which comprises a central region, a 5'-side region and a 3'-side region, wherein
   - the central region
      comprises at least 5 nucleotides independently selected from deoxyribonucleotides,
   - the 5'-side region
      comprises at least one nucleotide independently selected from the group consisting of deoxyribonucleotides and sugar moiety-modified nucleotides, and the 3'-terminal thereof is a sugar moiety-modified nucleotide, where the sugar moiety-modified nucleotide at the 3'-terminal binds to the central region, and selected from sugar moiety-modified nucleotides excluding 2'-3' bridged nucleotides and 3'-position modified non-bridged nucleotides, and
      does not contain an oligonucleotide strand constituted by at least four contiguous nucleotides which are independently selected from the group consisting of deoxyribonucleotides, 2'-3' bridged nucleotides and 3'-position modified non-bridged nucleotides; and
   - the 3'-side region
      comprises at least one nucleotide independently selected from the group consisting of deoxyribonucleotides and sugar moiety-modified nucleotides, and the 5'-terminal thereof is a sugar moiety-modified nucleotide, where the sugar moiety-modified nucleotide at the 5'-terminal binds to the central region, and selected from sugar moiety-modified nucleotides excluding 2'-3' bridged nucleotides and 3'-position modified non-bridged nucleotides, and
      does not contain an oligonucleotide strand constituted by at least four contiguous nucleotides which are independently selected from the group consisting of deoxyribonucleotides, 2'-3' bridged nucleotides and 3'-position modified non-bridged nucleotides.

      Above all, the antisense oligonucleotide of the following (3) is preferable.
(3) An antisense oligonucleotide which comprises a central region, a 5'-side region and a 3'-side region, wherein
   - the central region
      comprises at least 5 nucleotides independently selected from deoxyribonucleotides,
   - the 5'-side region
      comprises at least one nucleotide independently selected from sugar moiety-modified nucleotides, the sugar moiety-modified nucleotide at the 3'-end is bound to the central region, and selected from sugar moiety-modified nucleotides excluding 2'-3' bridged nucleotides and 3'-position modified non-bridged nucleotides,
   - the 3'-side region
      comprises at least one nucleotide independently selected from deoxyribonucleotides, the sugar moiety-modified nucleotide at the 5'-end is bound to the central region, and selected from sugar moiety-modified nucleotides excluding 2'-3' bridged nucleotides and 3'-position modified non-bridged nucleotides.

   In the above-mentioned (1), (2) and (3), the central region is preferably a gap region, the 5'-side region is preferably a 5'-wing region, and the 3'-side region is preferably a 3'-wing region. Also, a preferred embodiment of the 5'-side region and 3'-side region is the same as the 5'-side region and 3'-side region in the antisense oligonucleotide of the present invention. A preferred embodiment of the central region is the same as the central region in the antisense oligonucleotide of the present invention except that it does not contain sugar moiety-modified nucleotides selected from the group consisting of 2'-3' bridged nucleotides and 3'-position modified non-bridged nucleotides.
   As others, an antisense oligonucleotide (the so-called mixmer) of the following (4) may be mentioned.
(4) An antisense oligonucleotide which comprises at least 5 nucleotides independently selected from the group consisting of deoxyribonucleotides, ribonucleotides and sugar moiety-modified nucleotides, and
   does not contain an oligonucleotide strand constituted by at least four contiguous nucleotides which are independently selected from the group consisting of deoxyribonucleotides, 2'-3' bridged nucleotides and 3'-position modified non-bridged nucleotides.

The linking group that contains a non-nucleotide structure and the oligonucleotide can be bound by a common amidite method or H-phosphonate method. For example, after protecting one of the hydroxyl groups of a compound having two hydroxyl groups, the compound is derivatized to an amidite form by an amidite-forming reagent (for example, 2-cyanoethyl chloro(diisopropylamino)phosphinate, 2-cyanoethyl bis(diisopropylamino)phosphinate, and the like), or to an H-phosphonate form by an H-phosphonate reagent (for example, diphenyl phosphite, phosphorous acid, and the like), is capable of binding to an oligonucleotide, and deprotecting the above-mentioned protected hydroxyl group, and the nucleotide can be further extended by using a commercially available automatic nucleic acid synthesizer. The above-mentioned compound having two hydroxyl groups can be synthesized by using protection and deprotection reactions (for example, it can be referred to Protective Groups in Organic Synthesis, 4th Edition), oxidation reaction, reduction reaction, condensation reaction (oxidation reaction, reduction reaction and condensation reaction can be referred to, for example, Comprehensive Organic Transformations, 2nd Edition, written by R. C. Larock, Wiley-VCH (1999) and the like) and the like in combination, that are known for the persons of ordinary skill in the art, for example, from starting materials such as an amino acid, a carboxylic acid, a diol compound and the like. When the linking group containing a non-nucleotide structure has a functional group(s) (for example, an amino group, a hydroxy group or a thiol group) other than the above-mentioned two hydroxy groups, it can be efficiently extended by protecting these with a protective group (for example, it can be referred to Protective Groups in Organic Synthesis, 4th Edition) well known to the persons of ordinary skill in the art. Also, for synthesis of an oligonucleotide having a linking group containing a non-nucleotide structure, WO2012/017919, WO2013/103146, WO2013/133221, WO2015/099187, WO2016/104775 and the like can be referred to.

In addition, after synthesizing two oligonucleotides separately, linking groups that contains non-nucleotide structures are bonded. An example of the synthetic method is shown below. A partial structure having a functional group such as an amino group is bound to the 5'-end of the oligonucleotide by a method known to the persons of ordinary skill in the art (for example, 6-(trifluoroacetylamino)hexyl-[(2-cyanoethyl)-(N,N-diisopropyl)]-phosphoroamidite or the like is used), and a partial structure having a functional group such as an amino group is bound to the 3'-end of another oligonucleotide by a method known to the persons of ordinary skill in the art (for example, 2-((4,4'-dimethoxytrityl)oxymethyl)-6-fluorenylmethoxycarbonylamino-hexane-succinoyl-long chain alkylamino-CPG (GLEN RESEARCH, product number: 20-2958) and the like is used). Two functional groups possessed by the linking group that contains a non-nucleotide structure is converted into a desired functional group that reacts with the above-mentioned amino group and the like, whereby two oligonucleotides can be linked. For example, after converting two functional groups possessed by the linking group that contains a non-nucleotide structure into a carboxylic acid, an ester, an active ester (N-hydroxysuccinimidation and the like), an acid chloride, an activated carboxylic acid diester (4-nitrophenylated carboxylic acid diester and the like), isocyanate and the like, and they can be linked by the reaction under known N-carbonylation conditions. The above-mentioned N-carbonylation conditions can be referred to, for example, {(Comprehensive Organic Transformations Second Edition) 1999, (John Wiley & Sons, INC.)} and the like. The persons of ordinary skill in the art can protect one of the above-mentioned two functional groups, if necessary, and one oligonucleotide is bound to a linking group that contains a non-nucleotide structure and then deprotected, thereafter another oligonucleotide can be similarly bound to a linking group that contains a non-nucleotide structure.

The antisense oligonucleotide or a prodrug thereof include existing through their tautomerism and geometric isomerism, as well as those existing as a mixture thereof or a mixture of respective isomers. In addition, in the case of the presence of an asymmetric center or in the case of generating an asymmetric center as a result of isomerization, those of existing respective optical isomers thereof and mixtures of arbitrary ratios are also included. Also, in the case of a compound having two or more asymmetric centers, diastereomers are also present due to their respective optical isomers. The present invention includes all of these forms in optional ratio thereof. Also, the optical isomers can be obtained by the method well known for this purpose.

For example, when the antisense oligonucleotide or a prodrug thereof of the present invention contains a modified phosphodiester bond (for example, a phosphorothioate bond), and the phosphorus atom becomes an asymmetric atom, any forms of an oligonucleotide in which sterics of the phosphorus atom are controlled and an oligonucleotide in which sterics of the phosphorus atom are not controlled are included within the scope of the present invention.

The antisense oligonucleotide, a prodrug thereof or a pharmaceutically acceptable salt thereof of the present invention can exist in any crystalline form depending on the production conditions and can exist in any hydrate, and these crystalline forms, hydrates and mixtures thereof are also included within the scope of the present invention. In addition, it may also exist as a solvate containing an organic solvent such as acetone, ethanol, 1-propanol, 2-propanol and the like, and all of these forms are included within the scope of the present invention.

The antisense oligonucleotide or a prodrug thereof of the present invention can also be converted to a pharmaceutically acceptable salt or released from a formed salt if necessary. Examples of the pharmaceutically acceptable salt of the antisense oligonucleotide or a prodrug thereof may be mentioned, for example, a salt formed with an alkali metal (such as lithium, sodium and potassium), an alkaline earth metal (such as magnesium and calcium), ammonium, an organic base (such as triethylamine and trimethylamine), an amino acid (such as glycine, lysine and glutamic acid), an inorganic acid (such as hydrochloric acid, hydrobromic acid, phosphoric acid and sulfuric acid) or an organic acid (such as acetic acid, citric acid, maleic acid, fumaric acid, tartaric acid, benzenesulfonic acid, methanesulfonic acid and p-toluenesulfonic acid).

In particular, a partial structure represented by -P(=O)(OH)- may be converted to an anionic partial structure represented by -P(=O)(O⁻)- to form a salt with an alkali metal (lithium, sodium and potassium), an alkaline earth metal (magnesium and calcium) or ammonium. In addition, a partial structure represented by -P(=O)(SH)-, which forms a phosphorothioate bond, may be converted to an anionic partial structure represented by -P(=O)(S⁻)- to similarly form a salt with an alkali metal, an alkaline earth metal or ammonium. This is the same with regard to the other modified phosphodiester bond.

The antisense oligonucleotide or a prodrug thereof of the present invention can be produced by suitably selecting a method known to the persons of ordinary skill in the art. For example, the persons of ordinary skill in the art can be synthesized by designing the nucleotide sequence of the antisense oligonucleotide based on information of the nucleotide sequence of the target RNA using a commercially available automated nucleic acid synthesizer (such as that manufactured by Applied Biosystems, Beckman or GeneDesign Inc.). In addition, it can be also synthesized by a reaction using enzymes. As the above-mentioned enzymes, there may be mentioned polymerases, ligases and restriction enzymes, but the invention is not limited to these. That is, a method for producing the antisense oligonucleotide or a prodrug thereof according to the present embodiment can comprise a step for extending a nucleotide strand at the 3'-end or 5'-end.

A number of methods are known in the field of the art for bonding the functional molecule and the oligonucleotide, and can be referred to, for example, European Journal of Pharmaceutics and Biopharmaceutics, 2016, vol. 107, pp. 321-340, Advanced Drug Delivery Reviews, 2016, vol. 104, pp. 78-92, and Expert Opinion on Drug Delivery, 2014, vol. 11, pp. 791-822. For example, after bonding a functional molecule and a linker according to a known method, he resulting material is derived to an amidite with an amidite-forming reagent or derived to an H-phosphonate form with an H-phosphonate reagent followed by bonding to the oligonucleotide.

An antisense oligonucleotide or a prodrug thereof can be prepared by purifying the resulting oligonucleotide by reversed phase column chromatography and the like.

The antisense oligonucleotide or a prodrug thereof of the present invention can effectively control expression of a target gene. Accordingly, the present invention can provide, for example, a composition for controlling expression of a target gene based on an antisense effect, which contains the antisense oligonucleotide of the present invention as an effective ingredient. In particular, the antisense oligonucleotide or a prodrug thereof of the present invention can give high pharmacological efficacy by administering at a low concentration, and pharmaceutical compositions for the treatment, prevention and improvement of diseases associated with overexpression of a target gene such as metabolic diseases, tumors or infections can be also provided in several embodiments.

A composition containing the antisense oligonucleotide or a prodrug thereof of the present invention can be formulated according to a known pharmaceutical preparation method. For example, a composition containing the antisense oligonucleotide can be used either enterally (such as orally) or parenterally as a capsule, tablet, pill, liquid, powder, granule, fine granule, film-coated preparation, pellet, troche, sublingual preparation, chewed preparation, buccal preparation, paste, syrup, suspension, elixir, emulsion, coating preparation, ointment, plaster, poultice, transcutaneously absorbed preparation, lotion, inhalant, aerosol, injection preparation or suppository.

These preparations can be suitably combined with a pharmaceutically acceptable carrier or a carrier in the form of a food or beverage, specific examples of which include sterile water or physiological saline, vegetable oil, solvent, base, emulsifier, suspending agent, surfactant, pH adjuster, stabilizer, flavoring agent, fragrance, excipient, vehicle, preservative, binder, diluent, isotonic agent, analgesic, filler, disintegration agent, buffer, coating agent, lubricant, colorant, sweetener, thickening agents, corrective, solubilizing aid and other additives.

There are no particular limitations on the administration form of the composition containing the antisense oligonucleotide or a prodrug thereof of the present invention, and examples thereof include enteral (oral and the like) and parenteral administration. More preferably, there may be mentioned intravenous administration, intraarterial administration, intraperitoneal administration, subcutaneous administration, intradermal administration, intratracheal administration, rectal administration, intramuscular administration, intrathecal administration, intraventricular administration, transnasal administration and intravitreal administration, and administration by infusion.

There are no particular limitations on the disease able to be treated, prevented or ameliorated by using the antisense oligonucleotide or a prodrug thereof of the present invention, and examples thereof include metabolic diseases, circulatory diseases, tumors, infections, ophthalmic diseases, inflammatory diseases, autoimmune diseases, hereditary rare diseases, and diseases caused by expression of a gene. Specific examples include hypercholesterolemia, hypertriglyceridemia, spinal muscular atrophy, muscular dystrophy (such as Duchenne muscular dystrophy, myotonic dystrophy, congenital muscular dystrophy (such as Fukuyama-type congenital muscular dystrophy, Ullrich-type congenital muscular dystrophy, merosin-deficient congenital muscular dystrophy, integrin deficiency or Walker Warburg syndrome), Becker muscular dystrophy, limb-girdle muscular dystrophy, Miyoshi muscular dystrophy or facioscapulohumeral muscular dystrophy), Huntington's disease, Alzheimer's disease, transthyretin amyloidosis, familial amyloid cardiomyopathy, multiple sclerosis, Crohn's disease, inflammatory bowel disease, acromegaly, type 2 diabetes, chronic nephropathy, RS virus infection, Ebola hemorrhagic fever, Marburg virus, HIV, influenza, hepatitis B, hepatitis C, cirrhosis, chronic cardiac insufficiency, myocardial fibrosis, atrial fibrillation, prostate cancer, melanoma, breast cancer, pancreatic cancer, colorectal cancer, renal cell carcinoma, cholangiocarcinoma, cervical cancer, liver cancer, lung cancer, leukemia, non-Hodgkin's lymphoma, atopic dermatitis, glaucoma and age-related macular degeneration. The gene causing the above-mentioned disease can be set for the above-mentioned target gene corresponding to the type of the disease, and the above-mentioned expression control sequence (such as an antisense sequence) can be suitably set corresponding to the sequence of the above-mentioned target gene.

In addition to primates such as humans, a variety of other mammalian diseases can be treated, prevented, ameliorated by compositions comprising the antisense oligonucleotide or a prodrug thereof of the present invention. For example, although not limited thereto, various diseases of species of mammals, including cows, sheep, goats, horses, dogs, cats, guinea pigs and other bovines, ovines, equines, canines, felines and species of rodents such as mice can be treated. In addition, a composition containing the antisense oligonucleotide can also be applied to other species such as birds (such as chickens).

When a composition containing the antisense oligonucleotide or a prodrug thereof of the present invention is administered or fed to animals including humans, the administration dose or ingested amount thereof can be suitably selected depending on the age, body weight, symptoms or health status of the subject or the type of the composition (pharmaceuticals, food and drink) and the like, and the administration dose or ingested amount is preferably 0.0001 mg/kg/day to 100 mg/kg/day as the amount of the antisense oligonucleotide.

The antisense oligonucleotide or a prodrug thereof of the present invention can control expression of a target gene extremely effectively as well as can reduce in toxicity as compared to the conventional antisense oligonucleotide. Thus, a method for controlling expression of a target gene by an antisense effect more safety can be provided by administering the antisense oligonucleotide or a prodrug thereof of the present invention to animals, including humans. In addition, a method for treating, preventing or ameliorating various types of diseases associated with overexpression of a target gene can be also provided including providing a composition containing the antisense oligonucleotide or a prodrug thereof of the present invention to animals, including humans.

The following lists examples of preferable methods for using the antisense oligonucleotide of the present invention.

A method for controlling a function of a target RNA, comprising a step for contacting the antisense oligonucleotide or a prodrug thereof of the present invention with a cell.

A method for controlling a function of a target RNA in a mammal, comprising a step for administering a pharmaceutical composition containing the antisense oligonucleotide or a prodrug thereof of the present invention to the mammal.

In a mammal, a use of the antisense oligonucleotide or a prodrug thereof of the present invention for controlling a function of a target RNA.

In a mammal, a use of the antisense oligonucleotide or a prodrug thereof of the present invention for producing a drug for controlling a target RNA in a mammal.

A method for controlling an expression of a target gene, comprising a step for contacting the antisense oligonucleotide or a prodrug thereof of the present invention with a cell.

A method for controlling an expression of a target gene in a mammal, comprising a step for administering a pharmaceutical composition containing the antisense oligonucleotide or a prodrug thereof of the present invention to the mammal.

In a mammal, a use of the antisense oligonucleotide or a prodrug thereof of the present invention for controlling an expression of a target gene.

In a mammal, a use the antisense oligonucleotide or a prodrug thereof of the present invention for producing a drug for controlling an expression of a target gene.

Control of the function of a target RNA in the present invention refers to suppressing translation or regulating or converting a splicing function such as exon splicing that occurs by covering a portion of a target RNA due to hybridization by an antisense sequence portion, or suppressing a function of a target RNA by degrading the above-mentioned target RNA that can be generated as a result of recognition of a hybridized portion of an antisense sequence portion and a part of the target RNA.

The above-mentioned mammal is preferably a human.

The administration route is preferably enterally. As another embodiment, the administration route is parenterally.

The 2'-3' bridged nucleotide and 3'-position modified non-bridged nucleotide according to the embodiment of the present invention can be produced by the methods shown below in order, but the following producing method shows an example of general producing methods, and does not limit the producing method of the 2'-3' bridged nucleotide and 3'-position modified non-bridged nucleotide according to the present embodiment. As for the raw material compounds, when no specific producing method thereof is mentioned, commercially available compounds can be used, or they can be produced according to a known method or a method analogous thereto.

First, a general method for producing the following compound C, which is a representative three-membered ring 2'-3' bridged nucleotide, will be explained.

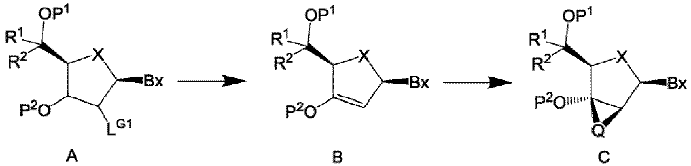

In the formula, P¹ and P² each independently a hydroxy protective group, L^{G1} represents a leaving group, -Q- represents -CR⁴R⁵-, -C(=O)-, -C(=S)-, or -C(=NR⁶)-, R⁴, R⁵, R⁶, and other symbols are the same as defined above.

Incidentally, the "leaving group" may be mentioned acetate (AcO), p-nitrobenzoate (PNBO), sulfonate (for example, methanesulfonate (mesylate: MsO), p-toluenesulfonate (tosylate: TsO), p-bromobenzenesulfonate (brosylate: BsO), p-nitrobenzenesulfonate (nosylate: NsO), fluoromethanesulfonate, difluoromethane-sulfonate, trifluoromethanesulfonate (triflate: TfO) and ethanesulfonate) and a halogen atom.

Compound A, which is a starting material, can be synthesized, for example, by converting 2' hydroxy of a ribonucleoside in which 3' and 5' hydroxy are protected into a leaving group. Conversion to a leaving group can be carried out, for example, by sulfonation (for example, methanesulfonation, p-toluenesulfonation) of an alcohol, and can be carried out by reacting chloromethanesulfonic acid or chloro-p-toluenesulfonic acid with a suitable base (for example, triethylamine or N,N-dimethyl-4-aminopyridine).

Compound A having various R¹ and R² can be synthesized, for example, from Compound A-1 described below by combining and using a protection/deprotection reaction (for example, the reaction described in the above-mentioned Protective Groups in Organic Synthesis 4th Edition), an oxidation reaction, and a reduction reaction (for example, it can be referred to Comprehensive Organic Transformations, 2nd Edition, written by R. C. Larock, Wiley-VCH (1999)) known to the persons of ordinary skill in the art.

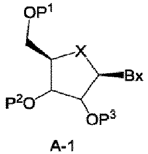

In the formula, P³ represents a hydroxy protective group, and other symbols are the same as defined above.

For example, in order to synthesize Compound A in which at least one of R¹ and R² is an alkyl group, first, hydroxy at the 3'-position is protected by the protection/deprotection reaction of the hydroxy to obtain a compound (Compound A-2) in which the hydroxy at the 5'-position is deprotected. Next, the hydroxy at the 5'-position of Compound A-2 is oxidized, and a desired R¹ can be introduced using an alkyl metal reagent or Grignard reagent corresponding to R¹. In addition, if necessary, the hydroxy at the 5'-position is once again oxidized, and a desired R² can be introduced using an alkyl metal reagent, metal hydride or Grignard reagent corresponding to R². By deprotecting the protected hydroxy at the 3'-position of the obtained compound, Compound A in which at least one of R¹ and R² is an alkyl group can be synthesized.

### (Synthesis of Compound B): Olefination

By removing the leaving group using an appropriate base (for example, DBU or sodium benzoate), an olefinated compound (Compound B) can be obtained. For example, there may be mentioned a method of reacting sodium benzoate in a solvent.

### (Synthesis of Compound C): Cyclization

When -Q- is -CR⁴R⁵-, a cyclized compound (C) can be synthesized by a generally known cyclopropanation reaction. For example, there may be mentioned a method of reacting diiodomethane which may be substituted by alkyl with diethylzinc in a solvent.

When -Q- is -C(=O)-, for example, a cyclized compound (C) can be synthesized by a method of reacting a protected hydroxydiiodomethane with diethylzinc, and then, deprotecting the protected hydroxy, and oxidizing it. When -Q- is -C(=S)-, a cyclized compound (Compound C) can be synthesized by thiocarbonylating the above-mentioned compound of -C(=O)- with a Lawesson's reagent or the like, and when -Q- is -C(=NR⁶)-, by iminating the above-mentioned compound where -Q- is - C(=O)- using an amine having a corresponding amino group.

Next, a general method for producing a representative four-membered ring, five-membered ring or six-membered ring 2'-3' bridged nucleotide will be explained.

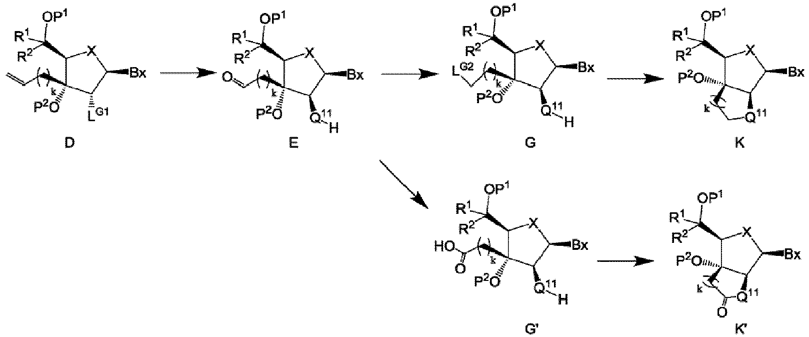

In the formula, P¹ and P² are each a hydroxy protective group, L^{G1} and L^{G2} are each independently leaving group, Q¹¹ is O, NH or NR⁶, H is a hydrogen atom, k is an integer of 0 to 3, and R⁶ and other symbols are the same as defined above.

Compound D, which is a starting material, can be synthesized by a method known for the persons of ordinary skill in the art such as a method described in Journal of the American Chemical Society, 1998, vol. 120, p. 5458, and Journal of the Chemical Society, Perkin Transaction 1, 1999, p. 2543.

Compound D having various R¹ and R² can be synthesized, for example, from Compound D-1 described below by combining and using a protection/deprotection reaction (for example, the reaction described in the above-mentioned Protective Groups in Organic Synthesis 4th Edition), an oxidation reaction, and a reduction reaction (for example, it can be referred to Comprehensive Organic Transformations, 2nd Edition, written by R. C. Larock, Wiley-VCH (1999)) known to the persons of ordinary skill in the art. Specific method is the same as the synthetic method of Compound A having various R¹ and R².

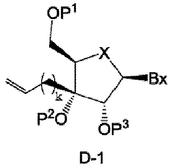

In the formula, P³ represents a hydroxy protective group, and other symbols are the same as defined above.

### (Synthesis of Compound E): Steric inversion substitution at 2'-position and carbonylation of olefin

By reacting the leaving group at the 2'-position with, for example, a base such as an aqueous sodium hydroxide solution and the like, in a solvent, a hydroxy compound in which hydroxy is positioned at the β-position of the 2'-position can be obtained. By reacting the leaving group at the 2'-position with an amine or ammonia which may have a substituent(s), in a solvent, an amino compound in which an amino group which may have a substituent(s) is positioned at the β-position of the 2'-position can be obtained. Or else, the amino compound can be also obtained by reduction with sodium azide.

Further, a carbonyl compound E can be obtained by dihydroxylizing the terminal olefin and oxidatively cleaving it with an oxidizing agent. For example, there may be mentioned a method in which, in a solvent, a catalytic amount of osmium tetroxide with sodium periodate is reacted.

### (Synthesis of Compound G): Reduction of carbonyl and conversion to leaving group

By using a suitable reducing agent (for example, sodium borohydride), carbonyl can be converted to hydroxy. The formed hydroxy is subjected to, for example, sulfonation (for example, methanesulfonation or p-toluenesulfonation), Compound G can be synthesized. For example, it can be carried out by reacting chloromethanesulfonic acid or chloro-p-toluenesulfonic acid with a suitable base (for example, triethylamine or N,N-dimethyl-4-aminopyridine).

### (Synthesis of Compound K): Cyclization

For example, in a solvent, by reacting with a suitable base (for example, sodium hydride), Compound K can be synthesized. Also, there is a case where cyclization may occur without adding a base.

### (Synthesis of Compound G'): Conversion of aldehyde to carboxylic acid

For example, in a solvent, by reacting with a suitable oxidizing agent (for example, chlorous acid), sodium dihydrogen phosphate and 2-methyl-2-butene, a carboxylic acid Compound G' can be obtained.

### (Synthesis of Compound K'): Cyclization

By condensing carboxy of Compound G' with hydroxy or amino by a known method, Compound K' can be synthesized. Also, after converting carboxy into an ester, an active ester (N-hydroxysuccinimidation or the like), an acid chloride and the like, it can be synthesized by a known condensation reaction.

In the process of obtaining Compound K from Compound E via Compound G, the reaction is carried out after protecting hydroxy or an amino group which may have a substituent(s), which is positioned at the β-position of the 2'-position, to obtain a compound (Compound G in which the 2'-position is protected) in which hydroxy or an amino group which may have a substituent(s) at the 2'-position of Compound G is protected, and the 2'-position of Compound G in which the 2'-position is protected is deprotected, and then, cyclization reaction may be carried out. The same applies to the process of obtaining Compound K' from Compound E via Compound G'.

Next, a general producing method of a representative 3'-position-modified non-bridged nucleotide is described. Synthesis of the 3'-position-modified non-bridged nucleotide can be referred to the method described in Journal of the Chemical Society, Perkin Transaction 1, 1998, p 1409 and the like.

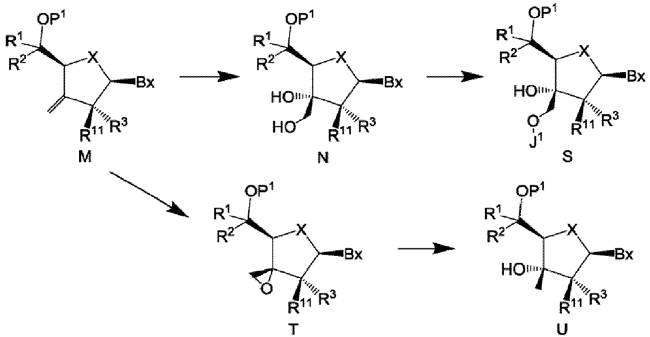

In the formula, P¹ is a hydroxy protective group, and other symbols are the same as defined above.

Compound M that is a starting material can be synthesized by a method known to the persons of ordinary skill in the art such as a method described in Journal of the Chemical Society, Perkin Transaction 1, 1998, p 1409 or the like.

Compound M having various R¹, R², R³ and R¹¹ can be synthesized, for example, from Compound M -1 or M-2 described below by combining and using a protection/deprotection reaction (for example, the reaction described in the above-mentioned Protective Groups in Organic Synthesis 4th Edition), an oxidation reaction, and a reduction reaction (for example, it can be referred to Comprehensive Organic Transformations, 2nd Edition, written by R. C. Larock, Wiley-VCH (1999) or the like) known to the persons of ordinary skill in the art. Specific method is the same as the synthetic method of Compound A having various R¹ and R².

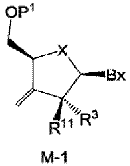

In the formula, the symbols in the formula are the same as defined above.

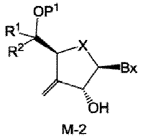

In the formula, the symbols in the formula are the same as defined above.

For example, Compound M in which at least one of R³ and R¹¹ is alkyl can be synthesized by firstly oxidizing hydroxy, and then, reducing it using an alkyl metal reagent, a Grignard reagent or the like.

### (Synthesis of Compound N): Dihydroxylation of olefin

Compound N can be synthesized by reacting to the 3'-position of olefin using a suitable dihydroxylation reagent in a solvent. Dihydroxylation can be carried out, for example, by using a catalytic amount of ruthenium chloride and a stoichiometric amount or more of sodium periodate.

### (Synthesis of Compound S): Alkylation of primary alcohol

Compound S can be synthesized by reacting a primary alcohol Compound N using a suitable alkylating reagent in a solvent. Alkylation can be carried out, for example, by reacting with an alkyl halide in the presence of a suitable base (for example, N, N-diisopropylethylamine).

### (Synthesis of Compounds T and U): Alkylation of primary alcohol

Compound U can be synthesized by epoxidization of Compound M and reduction of the obtained epoxy compound (Compound T). The synthetic method can be referred to a method described in Journal of the Chemical Society, Perkin Transaction 1, 1998, p 1409, or the like.

### EXAMPLES

Hereinafter, the present invention will be explained in more detail by referring to Examples and Comparative Examples, but the embodiments are not limited by the following Examples.

As an automatic nucleic acid synthesizer, nS-8II (manufactured by Gene Design Inc.) was used otherwise specifically described.

In the sequence notation (Tables 1, 2, 4, 5, 7, 8 and 10) in Examples, unless otherwise specifically described, "(L)" refers to LNA, alphabets with a small letter refers to a deoxyribonucleotide, alphabets with a capital letter (excluding the alphabets attached to the above-mentioned (L)) refers to a ribonucleotide, "^" refers to a phosphorothioate bond, "5" refers to that the base of the nucleotide is 5-methylcytosine, "(m)" refers to 2'-O-MOE modified nucleotide, and "FAM-" refers to that the 5'-end is labelled with 6-carboxyfluorescein. Also, Z₁ refers to a nucleotide structure represented by the following formula (Z₁).

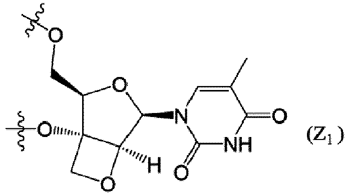

Also, Z₂ refers to a nucleotide structure represented by the following formula (Z₂).

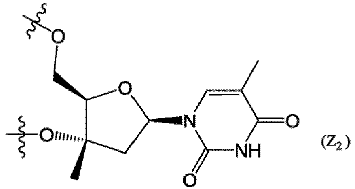

Labeling with 6-carboxyfluorescein at the 5'-end referred to that a moiety in which a hydroxy group is removed from one carboxy group of 6-carboxyfluorescein is bound to a moiety in which a hydrogen atom is removed from a hydroxy group at the 5'-end via a group represented by the formula: -P(=O)-O-(CH₂)₆-N(H)-. Incidentally, in the formula, a nitrogen atom is bound to a moiety in which a hydroxy group is removed from one carboxy group of 6-carboxyfluorescein, and a phosphorus atom is bound to a moiety in which a hydrogen atom is removed from a hydroxy group at the 5'-end.

### [Synthetic Example 1 of nucleotide]

(1R,2R,4R,5S)-1-(2-cyanoethoxy(diisopropylamino)phosphinoxy)-2-(4,4'-dimethoxytrityloxymethyl)-4-(thymin-1-yl)-3,6-dioxabicyclo-[3.2.0]heptane which is a 2'-O-3'-C-bridged modified nucleotide was synthesized by the method described in Journal of the American Chemical Society, 1998, 120, pp. 5458-5463.

### (Example 1, Comparative Example 1)

The antisense oligonucleotides described in Table 1 were prepared using an automatic nucleic acid synthesizer. The target gene is mouse Superoxide Dismutase-1 (SOD-1). The antisense oligonucleotide of Comparative Example 1 having no modification in the gap region has been reported to cause high toxicity due to the off-target effect (Nucleic Acids Research, 2016, 44, p 2093).

The molecular weights of the synthesized oligonucleotides were measured by MALDI-TOF-MASS. The results are shown in Table 1.

**[Table 1]**

| | Sequence (left side represents 5'-side and right side represents 3'-side) | Molecular weight actually measured value (M-H⁻) |
|---|---|---|
| Example 1 (SEQ.ID.NO:1) | T(L)^G(L)^A(L)^g^g^t^c^c^Z₁^g^c^a^c^T(L)^G(L)^G(L) | 5321.28 |
| Comparative Example 1 (SEQ.ID.NO:2) | T(L)^G(L)^A(L)^g^g^t^c^c^t^g^c^a^c^T(L)^G(L)^G(L) | 5350.65 |

### [Evaluation Example 1]

Cells of mouse brain endothelial cell line bEND. 3 were suspended in a DMEM medium containing 10% fetal bovine serum so as to be 5,000 cells/well, seeded in a 96-well plate (manufactured by Corning Inc., #3585), and cultured at 37°C under 5% CO₂ for about 24 hours. Each oligonucleotide of Table 1 was dissolved in a DMEM medium (test medium) containing 10% fetal bovine serum which contains 10 mM of calcium chloride so as to be the final concentration thereof of 10 nM, 30 nM, 100 nM, 300 nM or 1,000 nM, and after about 24 hours, the medium was replaced with a test medium and cultured (see Nucleic Acids Research, 2015, 43, p. e128). Further after 7 days, the cells were recovered and Total RNA was extracted from the cells using an RNeasy mini kit (manufactured by QIAGEN GmbH).

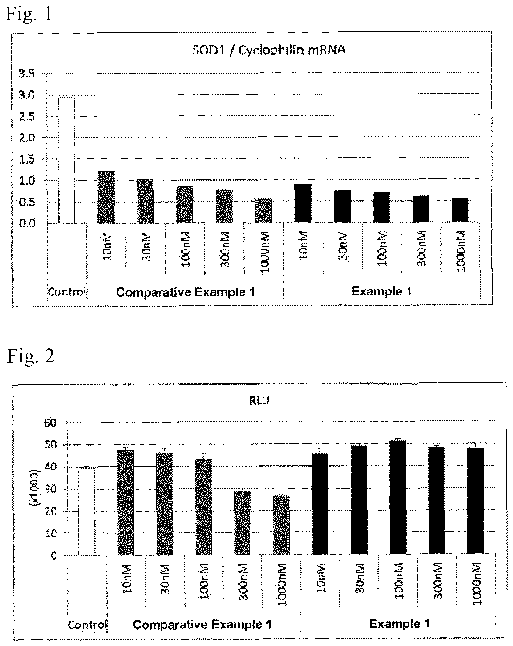

From Total RNA, cDNA was obtained using PrimeScript RT Master Mix (manufactured by TAKARA BIO INC.). Using the obtained cDNA and TaqMan (Registered Trademark) Gene Expression ID (manufactured by Applied Biosystems), real-time PCR was carried out by 7500 Real-Time PCR System (manufactured by Applied Biosystems) to quantify an amount of PTEN mRNA. In the real-time PCR, an amount of cyclophilin mRNA of housekeeping gene was also quantified at the same time, and the amount of PTEN mRNA to the amount of cyclophilin mRNA was evaluated as the PTEN expression level. Cells without addition of an oligonucleotide were used as controls. The results are shown in FIG. 1.

Incidentally, the primer used was TaqMan Gene Expression Assay (manufactured by Applied Biosystems), and Assay ID was as follows:
For mouse SOD-1 quantification: Mm01344233_g1
For mouse cyclophilin quantificaton: Mm0234230_g1

As clearly seen from FIG. 1, it was confirmed that the antisense oligonucleotide (Example 1) according to the present invention exhibits the same antisense effect as the antisense oligonucleotide having no modification in the gap region (Comparative Example 1).

### [Evaluation Example 2]

Cells of mouse brain endothelial cell line bEND. 3 were suspended in a DMEM medium containing 10% fetal bovine serum so as to be 5,000 cells/well, seeded in a 96-well plate (manufactured by Corning Inc., #3585), and cultured at 37°C under 5% CO₂ for about 24 hours. Each oligonucleotide of Table 1 was dissolved in a DMEM medium (test medium) containing 10% fetal bovine serum which contains 10 mM of calcium chloride so as to be the final concentration thereof of 10 nM, 30 nM, 100 nM, 300 nM or 1,000 nM, and after about 24 hours, the medium was replaced with a test medium and cultured (see Nucleic Acids Research, 2015, 43, p. e128). Further, 100 µL (CellTiter-Glo™ Luminescent Cell Viability Assay, manufactured by Promega Corporation) of an ATP reagent was added to the cell culture solution after 7 days to suspend therein, and after allowing to stand at room temperature for about 10 minutes, a luminescence intensity (RLU value) was measured by FlexStation 3 (manufactured by Molecular Devices Corp.), and the luminescence value of the culture medium alone was subtracted and the number of viable cells was measured as an average value of three points. Cells without addition of an oligonucleotide were used as controls.

As clearly seen from FIG. 2, it was confirmed that the antisense oligonucleotide (Example 1) according to the present invention had low cytotoxicity as compared with that of the antisense oligonucleotide having no modification in the gap region (Comparative Example 1). This is a result suggesting that the cytotoxicity caused by the off-target effect appeared in Comparative Example 1 is reduced by inserting a nucleic acid in which the 2'-position and the 3'-position are bridged in the gap region.

### [Evaluation Example 3]

RNAs (RNA (SOD-1)) complementary to the oligonucleotides of Example 1 and Comparative Example 1 which were labeled with 6-carboxyfluorescein at the 5'-end shown in Table 2 were synthesized. The molecular weight of RNA (SOD-1) was measured by MALDI-TOF-MASS. The measured value of the molecular weight was 5645.58 (M-H⁻).

**[Table 2]**

| | Sequence (left side represents 5'-side and right side represents 3'-side) |
|---|---|
| RNA(SOD-1) (SEQ.ID.NO:3) | FAM-CCAGUGCAGGACCUCA |

To water (150 µL) were added each oligonucleotide (150 pmol) in Table 1, RNA (450 pmol) in Table 2 and annealing buffer (200 mM KCl, 2 mM EDTA, pH=7.5) (60 µL), and the mixture was headed at 90°C for 2 minutes. Thereafter, the temperature was slowly lowered to 30°C, and maintained at this temperature. Solution A (750 mM KCl, 500 mM Tris-HCl, 30 mM MgCl₂, 100 mM dithiothreitol, pH=8.0) (30 µL) and recombinant RNase H derived from E.coli (manufactured by Wako Pure Chemical Industries, Ltd.) (1 unit) were added to the mixture, and the resulting mixture was reacted at 30°C for 2 hours. The enzyme was inactivated by transferring to an oil bath at 90°C and holding for 5 minutes, and cleavage activity of RNA was measured by reverse phase HPLC.

### (HPLC analytical conditions)

Eluent: Aqueous solution containing 0.1 M hexafluoroisopropyl alcohol and 8 mM triethylamine/methanol=95/5 (1 minute)→(14 minutes)→75/25 (3.5 minutes)
Flow rate: 1.0 mL/min
Column: WatersXBridge™ C18 2.5 µm, 4.6 mm × 75 mm
Column temperature: 60°C
Detection: Fluorescence (Em 518 nm, Ex 494 nm)

The results are shown in Table 3. In Table 3, the "conversion rate" indicates the ratio at which RNA (16mer) was cleaved, and represented by [100 - (RNA (16mer) ÷ (sum of area values of each peak) × 100)]. Also, the "number (mer) with bold-faced indication" represents cleaved RNA fragment(s), and is each represented by the number of nucleotides counted from the 5'-end. The "cleaved RNA area (%)" represents the area percentage (%) of each RNA fragment peak.

**[Table 3]**

| | Conversior rate (%) | Cleaved RNA area (%) | | | | |
|---|---|---|---|---|---|---|
| | | **8mer** | **9mer** | **1 0mer** | **11mer** | **12mer** |
| Comparative Example 1 | 97.2 | 80.1 | 9.7 | 4.0 | 3.0 | 3.2 |
| Example 1 | 92.6 | 92.0 | 0.1 | 0.5 | 0.1 | 7.2 |

With regard to Table 3, it is confirmed that there are almost no peak from 1mer to 7mer, and 13mer to 15mer. Incidentally, the 7mer to 11mer were separately prepared using an automatic nucleic acid synthesizer, and the molecular weight thereof was measured by MALDI-TOF-MASS. The actually measured value of the molecular weight was as follows.
11mer: 4095.60 (M-H⁻)
10mer: 3764.68 (M-H⁻)
9mer: 3420.65 (M-H⁻)
8mer: 3074.77 (M-H⁻)
7mer: 2746.23 (M-H⁻)
The retention times of those peaks under the above-mentioned HPLC analytical conditions 1 were confirmed. Other RNA fragments in Table 3 can be estimated from the retention time of the peak under the above-mentioned HPLC analytical conditions 1.

As clearly seen from Table 3, it was shown that the antisense oligonucleotide (Example 1) according to the present invention is improved in selectivity of the cleaved position in the region near to the modified position as compared with the antisense oligonucleotide having no modification in the gap region (Comparative Example 1). From the results of the above-mentioned Evaluation Examples 1 to 3, it was suggested that modification that improves the selectivity of the cleaved position reduces cytotoxicity.

### (Example 2, Comparative Example 2)

The antisense oligonucleotides described in Table 4 were prepared using an automatic nucleic acid synthesizer. The target gene is mouse coagulation factor XI (FXI). The antisense oligonucleotide of Comparative Example 2 having no modification at the gap region has been reported to cause high toxicity caused by the off-target effect (Nucleic Acids Research, 2016, 44, pp. 2093-2109).

The molecular weight of the synthesized oligonucleotides was measured by MALDI-TOF-MASS. The results are shown in Table 4.

**[Table 4]**

| | Sequence (left side represents 5'-side and right side represents 3'-side) | Molecular weight actually measured value (M-H⁻) |
|---|---|---|
| Example 2 (SEQ.ID.NO:4) | A(L)^T(L)^5(L)^t^g^t^g^c^a^Z₁^c^t^c^T(L)^5(L)^5(L) | 5263.22 |
| Comparative Example 2 (SEQ.ID.NO:5) | A(L)^T(L)^5(L)^t^g^t^g^c^a^t^c^t^c^T(L)^5(L)^5(L) | 5234.81 |

### [Evaluation Example 4]

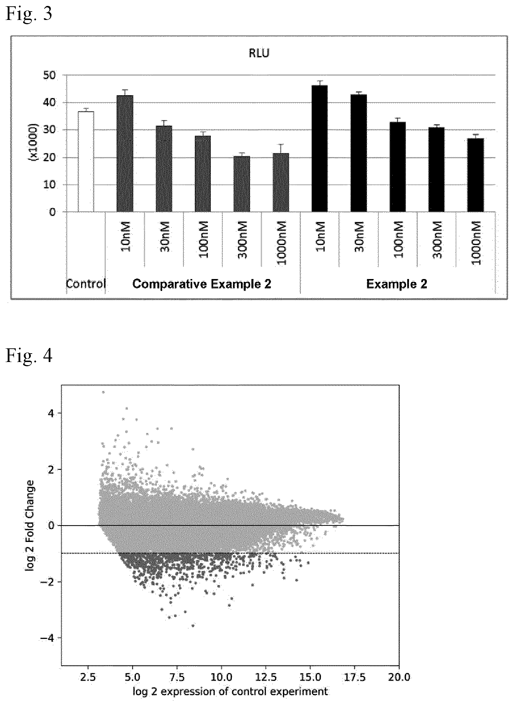

Using the same evaluation method as in Evaluation Example 2, the final concentration of each oligonucleotide in Table 4 was adjusted to 10 nM, 30 nM, 100 nM, 300 nM or 1000 nM, and the number of viable cells was measured. Cells without addition of an oligonucleotide were used as controls.
The results are shown in FIG. 3.

As clearly seen from FIG. 3, it was confirmed that the antisense oligonucleotide (Example 2) according to the present invention had low cytotoxicity as compared with that of the antisense oligonucleotide having no modification in the gap region (Comparative Example 2). This is a result suggesting that the cytotoxicity caused by the off-target effect appeared in Comparative Example 2 is reduced by inserting a nucleic acid in which the 2'-position and the 3'-position are bridged in the gap region.

### [Evaluation Example 5]

RNAs (RNA (FXI)) complementary to the oligonucleotides of Example 2 and Comparative Example 2 which were labeled with 6-carboxyfluorescein at the 5'-end shown in Table 5 were synthesized. The molecular weight of RNA (FXI) was measured by MALDI-TOF-MASS. The measured value of the molecular weight was 5773.54 (M-H⁻).

**[Table 5]**

| | Sequence (left side represents 5'-side and right side represents 3'-side) |
|---|---|
| RNA (FXI) (SEQ.ID.NO:6) | FAM-GGAGAGAUGCACAGAU |

The cleavage activity of RNA was measured using the same evaluation method as in Evaluation Example 3. However, the reaction time was made 1.5 hours.

The results are shown in Table 6. The indications in Table 6 are the same as those in Table 3.

**[Table 6]**

| | Conversion rate (%) | Cleaved RNA area (%) | | | | | | |
|---|---|---|---|---|---|---|---|---|
| | | **7mer** | **8mer** | **9mer** | **10mer** | **11mer** | **12mer** | **13mer** |
| Comparative Example 2 | 100.0 | 8.0 | 50.5 | 3.0 | 17.6 | 16.7 | 3.2 | 1.2 |
| Example 2 | 100.0 | 49.5 | 2.8 | 2.0 | 1.1 | 25.7 | 11.8 | 7.1 |

With regard to Table 6, it is confirmed that there are almost no peak from 1mer to 6mer, and 14mer to 15mer. Incidentally, the 6mer to 10mer were separately prepared using an automatic nucleic acid synthesizer, and the molecular weight thereof was measured by MALDI-TOF-MASS. The actually measured value of the molecular weight was as follows.
10mer: 3828.08 (M-H⁻)
9mer: 3521.67 (M-H⁻)
8mer: 3177.46 (M-H⁻)
7mer: 2873.46 (M-H⁻)
6mer: 2543.94 (M-H⁻)
The retention times of those peaks under the above-mentioned HPLC analytical conditions 1 were confirmed. Other RNA fragments in Table 3 can be estimated from the retention time of the peak under the above-mentioned HPLC analytical conditions 1.

As clearly seen from Table 6, it was shown that the antisense oligonucleotide (Example 2) according to the present invention is improved in selectivity of the cleaved position in the region near to the modified position (formation inhibition of 8mer and 10mer) as compared with the antisense oligonucleotide having no modification in the gap region (Comparative Example 2). From the results of the above-mentioned Evaluation Example 4 and Evaluation Example 5, it was suggested that modification that improves the selectivity of the cleaved position reduces cytotoxicity.

### (Example 3, Comparative Example 3)

The antisense oligonucleotides described in Table 7 were prepared using an automatic nucleic acid synthesizer. The target gene is human mutant type Huntington (muHTT), and is a site having an SNP mutated from wild type A to G (see Molecular Therapy - Nucleic Acids, 2017, 7, pp. 20-30).

**[Table 7]**

| | Sequence (left side represents 5'-side and right side represents 3'-side) | Molecular weight actually measured value (M-H⁻) |
|---|---|---|
| Example 3 (SEQ.ID.NO:7) | T(m)^A(L)^A(L)^a^t^t^g^Z₁^c^a^t^c^A(L)^5(L)^5(m) | 5049.35 |
| Comparative Example 3 (SEQ.ID.NO:8) | T(m)^A(L)^A(L)^a^t^t^g^t^c^a^t^c^A(L)^5(L)^5(m) | 5020.25 |

Mutant type RNA (mu-HTT, completely complement to the antisense oligonucleotide) labeled with 6-carboxyfluorescein at the 5'-end and wild type RNA (wt-HTT, containing a single base mismatch with the antisense oligonucleotide) described in Table 8 were synthesized.

**[Table 8]**

| | Sequence (left side represents 5'-side and right side represents 3'-side) |
|---|---|
| mu-HTT (SEQ.ID.NO:9) | FAM-GGUGAUGACAAUUUA |
| wt-HTT (SEQ.ID.NO:10) | FAM-GGUGAUGGCAAUUUA |

The molecular weights of the above-mentioned mu-HTT and wt-HTT were measured by MALDI-TOF-MASS. The actually measured values of the molecular weights were as follows.
mu-HTT: 5367.79 (M-H⁻)
wt-HTT: 5382.02 (M-H⁻)

### [Evaluation Example 6]

Using each oligonucleotide in Table 7 and each RNA in Table 8, the cleavage activity of RNA was measured using the same evaluation method as in Evaluation Example 3. However, the reaction time was made 0.5 hour.

The results are shown in Table 9. The indications in Table 9 are the same as those in Table 3.

**[Table 9]**

| | RNA | Conversion rate (%) | Cleaved RNA area (%) | | | | |
|---|---|---|---|---|---|---|---|
| | | | **8mer** | **9mer** | **10mer** | **11mer** | **12mer** |
| Comparative Example 3 | mu-HTT | 91.8 | 63.0 | 6.4 | 15.7 | 4.2 | 2.5 |
| Example 3 | mu-HTT | 88.7 | 81.4 | 0.0 | 4.9 | 0.3 | 2.1 |
| Comparative Example 3 | wt-HTT | 69.8 | 4.9 | 0.0 | 43.3 | 10.5 | 11.1 |
| Example 3 | wt-HTT | 39.4 | 9.2 | 0.0 | 4.3 | 1.2 | 23.1 |

With regard to Table 9, it is confirmed that there are almost no peak from 1mer to 7mer, and 13mer to 14mer. Incidentally, among the cleaved RNA fragments of mu-HTT, the 7mer to 13mer were separately prepared using an automatic nucleic acid synthesizer, and the molecular weight thereof was measured by MALDI-TOF-MASS. The actually measured value of the molecular weight was as follows.
13mer: 4730.56 (M-H⁻)
12mer: 4425.41 (M-H⁻)
11mer: 4117.98 (M-H⁻)
10mer: 3788.92 (M-H⁻)
9mer: 3460.61 (M-H⁻)
8mer: 3154.41 (M-H⁻)
7mer: 2825.87 (M-H⁻)
The retention times of those peaks under the above-mentioned HPLC analytical conditions 1 were confirmed. Other RNA fragments in Table 9 can be estimated from the retention time of the peak under the above-mentioned HPLC analytical conditions 1.

As clearly seen from Table 9, it was shown that selectivity of knockdown of mu-HTT to wt-HTT was higher in the antisense oligonucleotide (Example 3) according to the present invention (88.7÷39.4=2.3) as compared with the antisense oligonucleotide having no modification in the gap region (Comparative Example 2) (91.8÷69.8=1.3).

### [Evaluation Example 7]

Cells of mouse brain endothelial cell line bEND.3 were suspended in a DMEM medium containing 10% fetal bovine serum so as to be 40,000 cells/well, seeded in a 6-well plate (manufactured by Corning Inc., #3516), and cultured at 37°C under 5% CO₂ for about 24 hours. Each oligonucleotide of Table 1 was dissolved in a DMEM medium (test medium) containing 10% fetal bovine serum which contains 10 mM of calcium chloride so as to be the final concentration thereof of 3,000 nM, and after about 24 hours, the medium was replaced with a test medium and cultured (see Nucleic Acids Research, 2015, 43, p. e128). Further, after 24 hours, the cells were recovered and Total RNA was extracted from the cells using an RNeasy mini kit (manufactured by QIAGEN GmbH). Cells without addition of an oligonucleotide were used as controls.

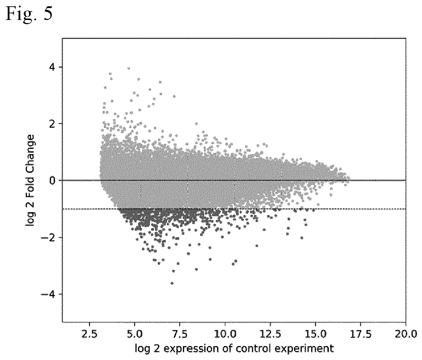

According to the conventional method, a complementary RNA fluorescently labeled with Cy3 [cyanine-3] was prepared from the Total RNA. The fluorescently labeled complementary RNA and SurePrint G3 Mouse Gene Expression 8x60K v2 (hybridized Agilent Technologies) were hybridized by the one-color protocol. The obtained signal data were analyzed by using GeneSpring software (manufactured by Agilent Technologies), fluctuations in gene expression levels relative to controls were comprehensively analyzed. The results of Comparative Example 1 are shown in FIG. 4, and the results of Example 1 are shown in FIG. 5. Incidentally, in FIG. 4 and FIG. 5, the horizontal axis (log 2 expression of control experiment) represents the expression level (log2) in the control specimen, and the vertical axis (log 2 fold change) represents the ratio (log2) of the change in expression relative to the control.

When the number of genes whose expression level was 50% or less was counted from FIG. 4 and FIG. 5, whereas the antisense oligonucleotide having no modification in the gap region (Comparative Example 1) was 760, the antisense oligonucleotide (Example 1) according to the present invention was 564. From these results, it was confirmed that the antisense oligonucleotide (Example 1) according to the present invention suppressed the off-target effect.

### [Synthetic Example 2 of nucleotide]

(2R,3S,5R)-2-(4,4'-dimethoxytrityloxymethyl)-3-methyl-5-(5-methyl-2,4-dioxo-3,4-dihydropyrimidin-1(2H)-yl)tetrahydrofuran-3-yl (2-cyanoethyl)phosphoramidite which is 3'-methyl-nucleotide was synthesized in accordance with the method described in Journal of the Chemical Society, Perkin Transactions 1, 1998, 120, pp. 5458-5463.

### (Example 4, Comparative Example 2)

The antisense oligonucleotide described in Table 10 was prepared using an automatic nucleic acid synthesizer. The target gene is mouse coagulation factor XI (FXI) as in Example 2 and Comparative Example 2.

The molecular weight of the synthesized oligonucleotide was measured by MALDI-TOF-MASS. The results are shown in Table 10.

**[Table 10]**

| | Sequence (left side represents 5'-side and right side represents 3'-side) | Molecular weight actually measured value (M-H⁻) |
|---|---|---|
| Example 4 (SEQ.ID.NO:11) | A(L)^T(L)^5(L)^t^g^t^g^c^a^Z₂^c^t^c^T(L)^5(L) ^5(L) | 5248.32 |

### [Evaluation Example 8]

The cleavage activity of RNA was measured using the same evaluation method as in Evaluation Example 5. The reaction time was made 1.5 hours.

The results are shown in Table 11. The indications in Table 11 are the same as those in Table 3.

**[Table 11]**

| | rate (%) | Conversion Cleaved RNA area (%) | | | | | | |
|---|---|---|---|---|---|---|---|---|
| | | **7mer** | **8mer** | **9mer** | **10mer** | **11mer** | **12mer** | **13mer** |
| Comparative Example 2 | 100.0 | 6.8 | 45.8 | 2.5 | 19.4 | 20.4 | 3.7 | 1.4 |
| example 4 | 100.0 | 0.0 | 10.5 | 1.3 | 3.1 | 51.4 | 14.7 | 19.0 |

With regard to Table 11, it is confirmed that there are almost no peak from 1mer to 6mer, 14mer and 15mer.

As clearly seen from Table 11, it was shown that the antisense oligonucleotide (Example 4) according to the present invention is improved in selectivity of the cleaved position in the region near to the modified position (formation inhibition of 8mer and 10mer) as compared with the antisense oligonucleotide having no modification in the gap region (Comparative Example 2).

### UTILIZABILITY IN INDUSTRY

The oligonucleotide of the present invention can suppress the off-target effect so that it is considered to be able to reduce toxicity, whereby it is useful as a pharmaceutical composition for the treatment or prevention of diseases associated with hyperfunction of a target RNA and/or overexpression of the target gene such as metabolic diseases, tumors or infections.

The disclosures of Japanese Patent Application 2018-052578 (filing date: March 20, 2018), Japanese Patent Application 2018-129296 (filing date: July 6, 2018) are incorporated herein by reference in their entirety. All documents, patent applications, and technical standards mentioned in the present description are also incorporated herein by reference to the same extent as if each individual document, patent application, and technical standard were specifically and individually noted to be incorporated by reference.

## Claims

1. An antisense oligonucleotide having a central region, a 5'-side region and a 3'-side region,
wherein
- the central region comprises
at least 5 nucleotides independently selected from the group consisting of deoxyribonucleotides, ribonucleotides and sugar moiety-modified nucleotides, contains at least one sugar moiety-modified nucleotide selected from the group consisting of a 2'-3' bridged nucleotide and 3'-position-modified non-bridged nucleotide, and a 3'-terminal and a 5'-terminal thereof being each independently a deoxyribonucleotide, ribonucleotide, 2'-3' bridged nucleotide or 3'-position-modified non-bridged nucleotide, and
contains at least one oligonucleotide strand constituted by at least four contiguous nucleotides which are independently selected from the group consisting of deoxyribonucleotides, 2'-3' bridged nucleotides and 3'-position-modified non-bridged nucleotides;
- the 5'-side region comprises
at least one nucleotide independently selected from the group consisting of deoxyribonucleotides, ribonucleotides and sugar moiety-modified nucleotides, and a 3'-terminal thereof being a sugar moiety-modified nucleotide, where the sugar moiety-modified nucleotide at the 3'-terminal binds to the central region, and is selected from the sugar moiety-modified nucleotides excluding a 2'-3' bridged nucleotide and 3'-position-modified non-bridged nucleotide, and
does not contain an oligonucleotide strand constituted by at least four contiguous nucleotides which are independently selected from the group consisting of deoxyribonucleotides, 2'-3' bridged nucleotides and 3'-position-modified non-bridged nucleotides; and
- the 3'-side region comprises
at least one nucleotide independently selected from the group consisting of deoxyribonucleotides, ribonucleotides and sugar moiety-modified nucleotides, and a 5'-terminal thereof being a sugar moiety-modified nucleotide, where the sugar moiety-modified nucleotide at the 5'-terminal binds to the central region, and is selected from the sugar moiety-modified nucleotides excluding a 2'-3' bridged nucleotide and 3'-position-modified non-bridged nucleotide, and
does not contain an oligonucleotide strand constituted by at least four contiguous nucleotides which are independently selected from the group consisting of deoxyribonucleotides, 2'-3' bridged nucleotides and 3'-position-modified non-bridged nucleotides.

2. The antisense oligonucleotide according to Claim 1, wherein
the central region comprises 5 to 15 nucleotides, and
the 5'-side region and the 3'-side region each independently comprise 1 to 7 nucleotides.

3. The antisense oligonucleotide according to Claim 1 or 2, wherein
the central region comprises 8 to 12 nucleotides,
the 5'-side region and the 3'-side region each independently comprise 2 to 5 nucleotides.

4. The antisense oligonucleotide according to any one of Claims 1 to 3, wherein
the 2'-3' bridged nucleotide contained in the central region is a nucleotide containing a partial structure represented by the following formula (I): 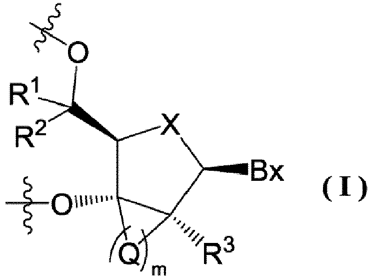
wherein m is 1, 2, 3 or 4,
Bx is a nucleic acid base moiety,
X is O or S,
-Q-'s are each independently -CR⁴R⁵-, -C(=O)-, -C(=S)-, -C(=NR⁶)-, -O-, -NH-, -NR⁶- or -S-,
when m is 2, 3 or 4, two adjacent -Q-'s may together form a group represented by the formula: -CR⁷=CR⁸-,
R¹, R², R³, R⁴ and R⁵ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano, J¹, J² and J³ are each independently a hydrogen atom or C1-C6 alkyl, Y is O, S or NJ⁴, and J⁴ is C1-C12 alkyl or an amino protective group;
R⁶ is C1-C12 alkyl or an amino protective group, and
R⁷ and R⁸ are each independently a hydrogen atom or C1-C6 alkyl.

5. The antisense oligonucleotide according to any one of Claims 1 to 3, wherein
the 3'-position-modified non-bridged nucleotide contained in the central region is a nucleotide containing a partial structure represented by the following formula (II): 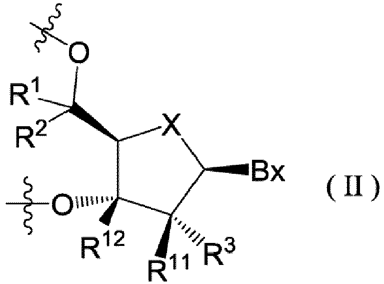
wherein Bx is a nucleic acid base moiety,
X is O or S,
R¹² is C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the above-mentioned substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano; R¹, R², R³ and R¹¹ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano;
J¹, J² and J³ are each independently a hydrogen atom or C1-C6 alkyl, Y is O, S or NJ⁴, and J⁴ is C1-C12 alkyl or an amino protective group.

6. The antisense oligonucleotide according to Claim 4, wherein
the 2'-3' bridged nucleotide contained in the central region is a nucleotide represented by the following formula (III): 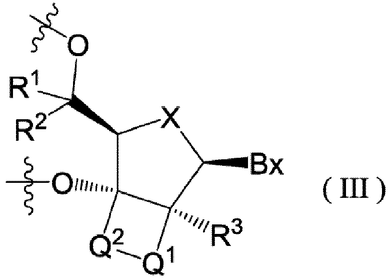
wherein Bx is a nucleic acid base moiety,
X is O or S,
-Q¹- and -Q²- are each independently -CR⁴R⁵-, -C(=O)-, -C(=S)-, -C(=NR⁶)-, -O-, -NH-, -NR⁶- or -S-, or
-Q¹-Q²- is -CR⁷=CR⁸-; and, wherein R⁷ and R⁸ are each independently a hydrogen atom or C1-C6 alkyl,
R¹, R², R³, R⁴ and R⁵ are each independently a hydrogen atom, C1-C6 alkyl, C2-C6 alkenyl, C2-C6 alkynyl, C1-C6 alkyl substituted by one or more substituents, C2-C6 alkenyl substituted by one or more substituents, C2-C6 alkynyl substituted by one or more substituents, acyl, acyl substituted by one or more substituents, amide substituted by one or more substituents, hydroxy, C1-C6 alkoxy, C1-C6 alkoxy substituted by one or more substituents, sulfanyl, C1-C6 alkylthio or C1-C6 alkylthio substituted by one or more substituents; where the substituents are each independently selected from the group consisting of a halogen atom, oxo, OJ¹, NJ¹J², SJ¹, azide, OC(=Y)J¹, OC(=Y)NJ¹J², NJ³C(=Y)NJ¹J² and cyano, J¹, J² and J³ are each independently a hydrogen atom or C1-C6 alkyl, Y is O, S or NJ⁴, and J⁴ is C1-C12 alkyl or an amino protective group;
R⁶ is C1-C12 alkyl or an amino protective group.

7. The antisense oligonucleotide according to Claim 6, wherein -Q¹- is -O-, -NH-, -NR⁶- or -S-, R⁶ is C1-C12 alkyl, and -Q²- is -CH₂-.

8. The antisense oligonucleotide according to Claim 6 or 7, wherein -Q¹- is -O-, and -Q²- is -CH₂-.

9. The antisense oligonucleotide according to any one of Claims 4 to 8, wherein R¹, R² and R³ are hydrogen atom.

10. The antisense oligonucleotide according to any one of Claims 4 to 9, wherein X is O.

11. The antisense oligonucleotide according to any one of Claims 1 to 10, wherein
the central region is a gap region,
the 5'-side region is a 5'-wing region, and
the 3'-side region is a 3'-wing region.

12. The antisense oligonucleotide according to any one of Claims 1 to 11, wherein
the sugar moiety-modified nucleotides contained in the 5'-side region and the 3'-side region are each independently selected from the group consisting of 2'-position-modified non-bridged nucleotide and 2',4'-BNA.

13. The antisense oligonucleotide according to Claim 12, wherein
the 2'-position-modified non-bridged nucleotide is at least one selected from the group consisting of 2'-O-methyl nucleotide, 2'-O-methoxyethyl (MOE) nucleotide, 2'-O-aminopropyl (AP) nucleotide, 2'-fluoronucleotide, 2'-O-(N-methylacetamido) (NMA) nucleotide and 2'-O-methylcarbamoylethyl (MCE) nucleotide.

14. The antisense oligonucleotide according to Claim 12, wherein
the 2',4'-BNA is at least one selected from the group consisting of LNA, cEt-BNA, ENA, BNA^{NC}, AmNA and scpBNA.

15. The antisense oligonucleotide according to any one of Claims 1 to 14, wherein
the antisense oligonucleotide contains a phosphorothioate bond.

16. The antisense oligonucleotide according to any one of Claims 1 to 15, which further comprises
a group derived from a functional molecule having at least one kind of a function selected from the group consisting of a labeling function, purifying function and delivering function to a target site.

17. The antisense oligonucleotide according to Claim 16, wherein
the functional molecule is selected from the group consisting of sugar, lipid, peptide and protein and their derivatives.

18. The antisense oligonucleotide according to Claim 16 or 17, wherein
the functional molecule is a lipid selected from the group consisting of cholesterol, tocopherol and tocotrienol.

19. The antisense oligonucleotide according to Claim 16 or 17, wherein
the functional molecule is a sugar derivative that interacts with an asialoglycoprotein receptor.

20. The antisense oligonucleotide according to Claim 16 or 17, wherein
the functional molecule is a peptide or a protein selected from the group consisting of receptor ligands and antibodies.

21. A prodrug which comprises the antisense oligonucleotide according to any one of Claims 1 to 20.

22. An oligonucleotide complex which comprises
(i) the antisense oligonucleotide according to any one of Claims 1 to 20, and
(ii) an oligonucleotide containing at least one ribonucleotide, and containing a region that hybridizes with the (i) antisense oligonucleotide.

23. An oligonucleotide which comprises
(i) the group derived from the antisense oligonucleotide according to any one of Claims 1 to 20, and
(ii) a group derived from an oligonucleotide containing at least one ribonucleotide, and containing a region that hybridizes with the antisense oligonucleotide of the (i), and the group derived from the antisense oligonucleotide of the (i), and the group derived from the oligonucleotides of the (ii) are linked.

24. An oligonucleotide complex which comprises
(iii) an oligonucleotide in which an oligonucleotide strand containing at least one ribonucleotide is linked to the group derived from the antisense oligonucleotide according to any one of Claims 1 to 20, and
(iv) an oligonucleotide containing an oligonucleotide strand which contains at least four contiguous nucleotides recognized by RNase H, and
the oligonucleotide strand containing at least one ribonucleotide of the (iii), and the oligonucleotide strand containing at least four contiguous nucleotides recognized by RNase H of the (iv) are hybridized.

25. An oligonucleotide which comprises
(iii) a group derived from an oligonucleotide in which an oligonucleotide strand containing at least one ribonucleotide is linked to a group derived from the antisense oligonucleotide according to any one of Claims 1 to 20, and
(iv) a group derived from an oligonucleotide containing an oligonucleotide strand which contains at least four contiguous nucleotides recognized by RNase H, and the group derived from the oligonucleotide of the (iii) and the group derived from the oligonucleotide of the (iv) are linked, and
the oligonucleotide strand containing at least one ribonucleotide of the above-mentioned (iii) and the oligonucleotide strand which contains at least four contiguous nucleotides recognized by RNase H of the above-mentioned (iv) are hybridized.

26. A pharmaceutical composition which comprises the antisense oligonucleotide according to any one of Claims 1 to 25, the prodrug according to Claim 21, the oligonucleotide complex according to Claim 22 or 24 or the oligonucleotide according to Claim 23 or 25, and a pharmacologically acceptable carrier.

27. A method for controlling a function of a target RNA which comprises a step of contacting the antisense oligonucleotide according to any one of Claims 1 to 20, the prodrug according to Claim 21, the oligonucleotide complex according to Claim 22 or 24 or the oligonucleotide according to Claim 23 or 25 with a cell.

28. A method for controlling a function of a target RNA in a mammal, which comprises a step of administering the pharmaceutical composition according to Claim 26 to the mammal.

29. A method for controlling development of a target gene which comprises a step of contacting the antisense oligonucleotide according to any one of Claims 1 to 20, the prodrug according to Claim 21, the oligonucleotide complex according to Claim 22 or 24 or the oligonucleotide according to Claim 23 or 25 with a cell.

30. A method for controlling development of a target gene in a mammal, which comprises a step of administering the pharmaceutical composition according to Claim 26 to the mammal.

31. A method for producing the antisense oligonucleotide according to any one of Claims 1 to 20 or the prodrug according to Claim 21 which comprises using a nucleotide selected from the group consisting of 2'-3' bridged nucleotide and 3'-position-modified non-bridged nucleotide.
